(19) Europäisches Patentamt
European Patent Office
Office européen des brevets

(11) Publication number: **0 549 857 A1**

(12) **EUROPEAN PATENT APPLICATION**

(21) Application number: **92116759.9**

(22) Date of filing: **30.09.92**

(51) Int. Cl.[5]: **C07D 401/04**, A61K 31/40, C07D 471/04, C07D 498/06

(30) Priority: **31.12.91 KR 9125886**

(43) Date of publication of application: **07.07.93 Bulletin 93/27**

(84) Designated Contracting States: **PT**

(71) Applicant: **KOREA RESEARCH INSTITUTE OF CHEMICAL TECHNOLOGY**
**100, Jang-Dong, Yuseong-Ku**
**Daejeon, 305-606(KR)**

(72) Inventor: **Kim, Wan Joo**
**383-7 Dorhyong-dong**
**Yuseng-ku, Daejeon 305-606(KR)**
Inventor: **Park, Myung Hwan**
**431 Dorhyong-dong**
**Yuseong-ku, Daejeon 305-343(KR)**
Inventor: **Ha, Jae Du**
**100 Jang-dong**
**Yuseong-ku, Daejeon 305-606(KR)**
Inventor: **Baik, Kyong Up**
**39-35 Gajang-dong**
**Seo-ku, Daejeon 302-181(KR)**
Inventor: **Lee, Tae Suk**
**Samchang APT. 1-203**
**Gajang-dong, Soe-ku Daejeon 302-181(KR)**
Inventor: **Park, Tae Ho**
**431-6 Dorhong-dong**
**Yuseong-ku, Daejeon 305-606(KR)**
Inventor: **Nam, Keun Soo**
**57-50 Gajang-dong**
**Seo-ku, Daejeon 302-181(KR)**
Inventor: **Kim, Bong Jin**
**405-16 Kung-dong**
**Yuseong-ku, Daejeon 305-606(KR)**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80 (DE)**

(54) **Antibacterial quinolone carboxylic acid derivatives.**

(57) The present invention relates to certain novel quinolone compounds of the present invention represented by the following formula(I) and novel processes for preparing same.

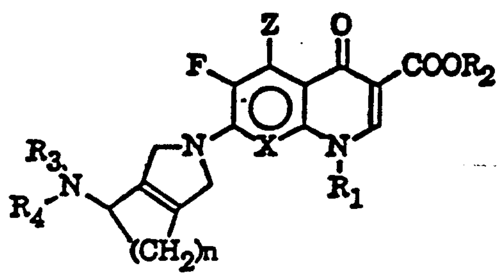

(I)

wherein:

X is a nitrogen atom or a C-Y group wherein Y is a hydrogen, fluorine, chlorine or bromine atom or a methoxy or methyl group;

$R_1$ is a $C_{1-6}$ alkyl group optionally substituted with a halogen atom or a hydroxy radical, an alkenyl group, a $C_{3-6}$ cycloalkyl group, a phenyl group substituted with a halogen atom or a divalent

EP 0 549 857 A1

group of $-OCH_2{}^*CH(CH_3)-$, $-SCH_2{}^*CH_2-$or $-SCH_2{}^*CH(CH_3)-$ which forms an oxazine or thiazine ring together with the nitrogen atom to which $R_1$ is attached and with X wherein X is C-Y;

| | |
|---|---|
| $R_2$ is | a hydrogen atom, a carboxy protecting group or a pharmaceutically acceptable metal or organic cation; |
| $R_3$ and $R_4$, | which may be the same or different, are a hydrogen atom, a lower alkyl group, an acyl group or a nitrogen protecting group metabolizable in vivo; |
| Z is | a hydrogen or halogen atom, an amino, hydroxy or methyl group; and |
| n is | 1 to 3. |

## Field of the Invention

The present invention relates to novel quinolone carboxylic acid derivatives, and pharmaceutically acceptable salts thereof, substituted at 7-position of the quinolone or at 10-position of the pyridobenzoxazine which possess a broad spectrum of potent anti-bacterial activities; and to novel processes for preparing these compounds.

## Description of the Prior Art

Quinolones such as enoxacin, norfloxcain, ofloxacin, ciprofloxacin, tosufloxacin and the like have been commercially available for sometime. However, most of these prior art materials are known to have relatively weak anti-bacterial potency especially against Gram-positive bacteria; and resistant microorganisms against these drugs have been found to exist.

Therefore, needs have existed for the development of new medicinal compounds which possess a broad scope of potent anti-bacterial activities and are effective against quinolone-resistant microorganisms including clinically important methicillin resistant Staphylococcus aureus(MRSA).

## Summary of the Invention

Unexpectedly, the present inventors have discovered that certain quinolone carboxylic acid derivatives substituted at 7-position of the quinolone or at 10-position of the pyridobenzoxazine meet the above requirements.

Accordingly, the present invention primarily pertains to said novel quinolone compounds ad pharmaceutically acceptable salts thereof; and novel processes for preparing these compounds.

## Detailed Description of the Invention

The novel quinolone compounds of the present invention can be represented by the following formula-(I):

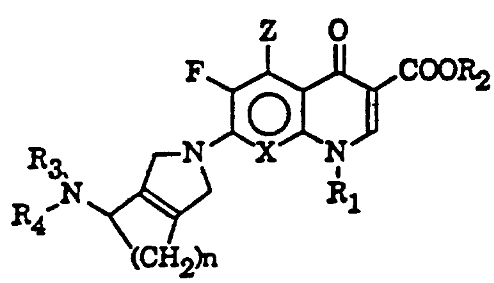

(I)

wherein:

| | |
|---|---|
| X is | a nitrogen atom or a C-Y group wherein Y is a hydrogen, fluorine, chlorine or bromine atom or a methoxy or methyl group; |
| $R_1$ is | a $C_{1-6}$ alkyl group optionally substituted with a halogen atom or a hydroxy radical, an alkenyl group, a $C_{3-6}$ cycloalkyl group, a phenyl group substituted with a halogen atom or a divalent group of $-OCH_2{}^*CH(CH_3)-$, $-SCH_2{}^*CH_2-$or $-SCH_2{}^*CH(CH_3)-$ which forms an oxazine or thiazine ring together with the nitrogen atom to which $R_1$ is attached and with X wherein X is C-Y; |
| $R_2$ is | a hydrogen atom, a carboxy protecting group or a pharmaceutically acceptable metal or organic cation; |
| $R_3$ and $R_4$, | which may be the same or different, are a hydrogen atom, a lower alkyl group, an acyl group or a nitrogen protecting group metabolizable in vivo; |
| Z is | a hydrogen or halogen atom, an amino, hydroxy or methyl group; and |
| n is | 1 to 3. |

The compounds of formula(I) encompass their acid or base addition salts and hydrates.

In the definition of $R_1$, said $C_{1-6}$ alkyl group is preferably a methyl, ethyl, n-propyl, isobutyl, t-butyl, n-pentyl or n-hexyl group, more preferably, an ethyl or t-butyl group;

said $C_{1-6}$ haloalkyl group is preferably a $C_{2-4}$ alkyl group substituted with a fluorine atom, e.g., 2-fluoroethyl group;

said $C_{1-6}$ hydroxyalkyl group is preferably a $C_{2-4}$ hydroxyalkyl group, e.g., 2-hydroxyethyl group;

said $C_{3-6}$ cycloalkyl group is preferably a cyclopropyl, cyclobutyl, cyclopentyl or cyclohexyl group, more preferably, a cyclopropyl group;

said alkenyl group is preferably a vinyl, isopropenyl, propenyl or isobutenyl group;

said phenyl group substituted with a halogen atom is preferably a phenyl group substituted with one or two fluorine atoms, more preferably, 4-fluorophenyl or 2,4-difluorophenyl group; and

the stereochemical configuration of the chiral atom of $-OCH_2{}^*CH(CH_3)-$ or $-SCH_2{}^*CH(CH_3)-$ group may be (R), (S) or a mixture thereof.

In the definition of $R_2$, said carboxy protecting group is an ester moiety which can be readily, e.g., hydrolyzed to produce a free carboxylic acid, e.g., a lower alkyl group such as methyl, ethyl, n-propyl or t-butyl group, a lower alkanoyloxy-lower alkyl group such as acetoxymethyl or pivaloyloxymethyl group, a lower alkoxycarbonyloxy-lower alkyl group such as methoxycarbonyloxymethyl or 1-ethoxycarbonyloxyethyl group, a lower alkoxymethyl group such as methoxymethyl group, a di(lower alkyl)amino-lower alkyl group such as 1-dimethylaminoethyl group, or a 4-methylene-5-methyl-1,3-dioxolene-2-one group; and

said pharmaceutically acceptable metal or organic cation is preferably a cation of alkaline metal or alkaline earth metal such as sodium, potassium, silver, calcium or magnesium cation or an organic cation such as tertiary or quarternary $C_{1-4}$ alkyl ammonium cation.

In the definition of $R_3$ ad $R_4$, said lower alkyl group is preferably a methyl, ethyl, propyl or butyl group, more preferably, a methyl or ethyl group;

said acyl group is preferably an acetyl, propionyl or benzoyl group; and

said nitrogen protecting group metabolizable in vivo is preferably a formyl, alkoxycarbonyl, alkylcarbonylmethylene, alkoxycarbonylmethylene or 4-methylene-5-methyl-1,3-dioxolene-2-one group.

In the definition of Z, said halogen atom is preferably a fluorine atom.

In the definition of n, n is preferably 2 or 3.

The stereochemical configuration of azabicycloamine substituted at 7-position of the compounds of formula(I) may be(R), (S) or a mixture thereof.

Among the compounds of the present invention, preferred are: 7-[6-amino-3-azabicylo[3,3,0]oct-1(5)ene-3-yl]-1-cyclopropyl-6,8-difluoro-1,4-dihydro-4-oxoquinoline-3-carboxylic acid or its hydrochloride salt or its lactic acid salt, 1-cyclopropyl-6,8-difluoro-7-[6-methylamino-3-azabicyclo[3,3,0]oct-1(5)ene-3-yl]-1,4-dihydro-4-oxoquinoline-3-carboxylic acid, 7-[6-amino-3-azabicyclo [3,3,0]oct-1(5)ene-3-yl]-1-cyclopropyl-8-chloro-6-fluoro-1,4-dihydro-4-oxoquinoline-3-carboxylic acid or its hydrochloride salt or its lactic acid salt, 7-[6-methylamino-3-azabicyclo[3,3,0]oct-1(5)ene-3-yl]-8-chloro-1-cyclopropyl-6-fluoro-1,4-dihydro-4-oxoquinoline-3-carboxylic acid, 7-[6-amino-3-azabicyclo[3,3,0]oct-1(5)ene-3-yl]-8-bromo-1-cyclopropyl-6-fluoro-1,4-dihydro-4-oxoquinoline-3-carboxylic acid, 7-[6-amino-3-azabicyclo[3,3,0]oct-1(5)ene-3-yl]-1-cyclopropyl-6-fluoro-8-methoxy-1,4-dihydro-4-oxoquinoline-3-carboxylic acid, 1-cyclopropyl-6-fluoro-7-[6-methylamino-3-azabicyclo[3,3,0]oct-1(5)ene-3-yl]-1,4-dihydro-4-oxo-1,8-naphthyridine-3-carboxylic acid, 7-[6-amino-3-azabicyclo[3,3,0]oct-1(5)ene-3-yl]-6,8-difluoro-1-ethyl-1,4-dihydro-4-oxoquinoline-3-carboxylic acid, 7-[6-amino-3-azabicyclo[4,3,0]non-1(6)ene-3-yl]-1-cyclopropyl-6,8-difluoro-1,4-dihydro-4-oxoquinoline-3-carboxylic acid, 7-[6-amino-3-azabicyclo[3,3,0]oct-1(5)ene-3-yl]-5-amino-1-cyclopropyl-6,8-difluoro-1,4-dihydro-4-oxoquinoline-3-carboxylic acid, 7-[6-methylamino-3-azabicyclo[3,3,0]oct-1(5)ene-3-yl]-5-amino-1-cyclopropyl-6,8-difluoro-1,4-dihydro-4-oxoquinoline-3-carboxylic acid, 7-[6-amino-3-azabicyclo[3,3,0]oct-1(5)-ene-3-yl]-1-(2,4-difluorophenyl-6,8-difluoro-1,4-dihydro-4-oxoquinoline-3-carboxylic acid, 7-[6-methylamino-3-azabicyclo[3,3,0]oct-1(5)ene-3-yl]-1-(2,4-difluorophenyl)-6,8-difluoro-1,4-dihydro-4-oxoquinoline-3-carboxylic acid, 7-[6-amino-3-azabicyclo[3,3,0]oct-1(5)ene-3-yl]-1-cyclopropyl-6-fluoro-1,4-dihydro-4-oxo-1,8-naphthyridine-3-carboxylic acid, 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxylic acid, 9-fluoro-7-[6-amino-3-azabicyclo[3,3,0]oct-1(5)-ene-3-yl]-3-(S)-methyl-7-oxo-2,3-dihydro-7H-pyrido[1,2,3-de]-1,4-benzoxazine-6-carboxylic acid, 9-fluoro-7-[6-methylamino-3-azabicyclo[3,3,0] oct-1(5)ene-3-yl]-3-(S)methyl-7-oxo-2,3-dihydro-7H-pyrido[1,2,3-de]-1,4-benzoxazine-6-carboxylic acid, 7-[6-amino-3-azabicyclo[3,3,0] oct-1(5)ene-3-yl]-1-ethyl-6-fluoro-1,4-dihydro-4-oxoquinoline-3-carboxylic acid, 7-[6-methyl-amino-3-azabicyclo[3,3,0]oct-1(5)ene-3-yl]-1-ehtyl-6-fluoro-1,4-dihydro-4-oxoquinoline-3-carboxylic acid, 7-[6-amino-3-azabicyclo[3,3,0]oct-1(5)ene-3-yl]-1-ethyl-6-fluoro-1,4-dihydro-4-oxoquinoline-3-carboxylic acid, 7-[6-methylamino-3-azabicyclo[3,3,0]oct-1(5)ene-3-yl]-1-ethyl-6-fluoro-8-chloro-1,4-dihydro-4-oxoquinoline-3-carboxylic acid, 7-[6-amino-3-azabicyclo [3,3,0]oct-1(5)ene-3-yl-1-ethyl-5-amino-6,8-difluoro-1,4-dihydro-4-oxoquinoline-3-carboxylic acid, 7-[6-methylamino-3-azabicyclo [3,3,0]oct-1(5)ene-3-yl]-1-ethyl-5-amino-6,8-difluoro-1,4-dihydro-4-oxoquinoline-3-carboxylic acid, 7-[6-amino-3-azabicyclo[3,3,0] oct-1(5)ene-3-yl]-1-(t-butyl)-6,8-difluoro-1,4-dihydro-4-oxoquinoline-3-carboxylic

acid, 7-[6-methylamino-3-azabicyclo[3,3,0]oct-1(5)ene-3-yl]-1-(t-butyl)-6,8-difluoro-1,4-dihydro-4-oxoquinoline-3-carboxylic acid, 7-[6-amino-3-azabicyclo[3,3,0]oct-1(5)ene-3-yl]-1-(t-butyl)-6-fluoro-8-chloro-1,4-dihydro-4-oxoquinoline-3-carboxylic acid, 7-[6-methylamino-3-azabicyclo[3,3,0]oct-1(5)ene-3-yl]-1-(t-butyl)-6-fluoro-8-chloro-1,4-dihydro-4-oxoquinoline-3-carboxylic acid, 7-[6-amino-3-azabicyclo[3,3,0]oct-1(5)-ene-3-yl]-1-(4-fluorophenyl)-6-fluoro-1,4-dihydro-4-oxoquinoline-3-carboxylic acid, 7-[6-amino-3-azabicyclo-[3,3,0]oct-1(5)ene-3-yl]-1-(4-fluorophenyl)-6,8-difluoro-1,4-dihydro-4-oxoquinoline-3-carboxylic acid, 7-[6-methylamino-3-azabicyclo[3,3,0]oct-1(5)ene-3-yl]-1-(4-fluorophenyl)-6,8-difluoro-1,4-dihydro-4-oxoquinoline-3-carboxylic acid, 7-[6-amino-3-azabicyclo [3,3,0]oct-1(5)ene-3-yl]-1-ethyl-6-fluoro-1,4-dihydro-4-oxo-1,8-napthyridine-3-carboxylic acid, 7-[6-methylamino-3-azabicyclo[3,3,0] oct-1(5)ene-3-yl]-1-ethyl-6-fluoro-1,4-dihydro-4-oxo-1,8-naphthyridine-3-carboxylic acid, 7-[6-amino-3-azabicyclo[3,3,0]oct-1(5)ene-3-yl]-1-(t-butyl)-6-fluoro-1,4-dihydro-4-oxoquinoline-3-carboxylic acid, 7-[6-methylamino-3-azabicyclo[3,3,0]oct-1(5)-ene-3-yl]-1-(t-butyl)-6-fluoro-1,4-dihydro-4-oxoquinoline-3-carboxylic acid, 7-[6-acetamino-3-azabicyclo-[3,3,0]oct-1(5)ene-3-yl]-1-cyclopropyl-6,8-difluoro-1,4-dihydro-4-oxoquinoline-3-carboxylic acid and 7-[6-t-butoxycarbonylamino-3-azabicyclo[3,3,0]oct-1(5)ene-3-yl]-1-cyclopropyl-6,8-difluoro-1,4-dihydro-4-oxoquinoline-3-carboxylic acid methoxymethyl ester.

More preferred are: 7-[6-amino-3-azabicyclo[3,3,0]oct-1(5) ene-3-yl]-1-cyclopropyl-6,8-difluoro-1,4-dihydro-4-oxoquinoline-3-carboxylic aicd or its hydrochloric acid salt or its lactic acid salt, 1-cyclopropyl-6,8-difluoro-7-[6-methylamino-3-azabicyclo[3,3,0] oct-1(5)ene-3-yl]-1,4-dihydro-4-oxoquinoline-3-carboxylic acid, 7-[6-amino-3-azabicyclo[3,3,0]oct-1(5)ene-3-yl]-1-cyclopropyl-8-chloro-6-fluoro-1,4-dihydro-4-oxoquinoline-3-carboyxlic acid or its hydrochloric acid salt or its lactic acid salt, 7-[6-methylamino-3-azabicyclo[3,3,0]oct-1-(5)ene-3-yl]-8-chloro-1-cyclopropyl-6-fluoro-1,4-dihydro-4-oxoquinoline-3-carboxylic acid, 7-[6-amino-3-azabicyclo [3,3,0]oct-1(5)ene-3-yl]-1-cyclopropyl-6-fluoro-8-methoxy-1,4-dihydro-4-oxoquinoline-3-carboxylic acid, 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxylic acid, 7-[6-amino-3-azabicyclo [3,3,0]oct-1(5)ene-3-yl]-5-amino-1-cyclopropyl-6,8-difluoro-1,4-dihydro-4-oxoquinoline-3-carboxylic acid, 7-[6-amino-3-azabicyclo [3,3,0]oct-1(5)ene-3-yl]-1-cyclopropyl-6-fluoro-1,4-dihydro-4-oxo-1,8-naphthyridine-3-carboxylic acid, 9-fluoro-10-[6-amino-3-azabicyclo[3,3,0]oct-1(5)ene-3-yl]-3-(S)-methyl-7-oxo-2,3-dihydro-7H-pyrido[1,2,3,-de]-1,4-benzoxazine-6-carboxylic acid, 9-fluoro-10-[6-methylamino-3-azabicyclo[3,3,0]oct-1(5)ene-3-yl]-3-(S)-methyl-7-oxo-2,3-dihydro-7H-pyrido[1,2,3-de]-1,4-benzoxazine-6-carboxylic acid, 7-[6-amino-3-azabicylo[3,3,0]oct-1(5)ene-3-yl]-1-(t-butyl)-6-fluoro-1,4-dihydro-4-oxoquinoline-3-carboxylic acid, 7-[6-t-butoxycarbonylamino-3-azabicyclo[3,3,0]oct-1(5)ene-3-yl]-1-cyclopropyl-6,8-difluoro-1,4-dihydro-4-oxoquinoline-3-carboxylic acid.

The quinolone compounds of the present invention represented by formula(I) and pharmaceutically acceptable salts thereof can be prepared as follows.

Process A

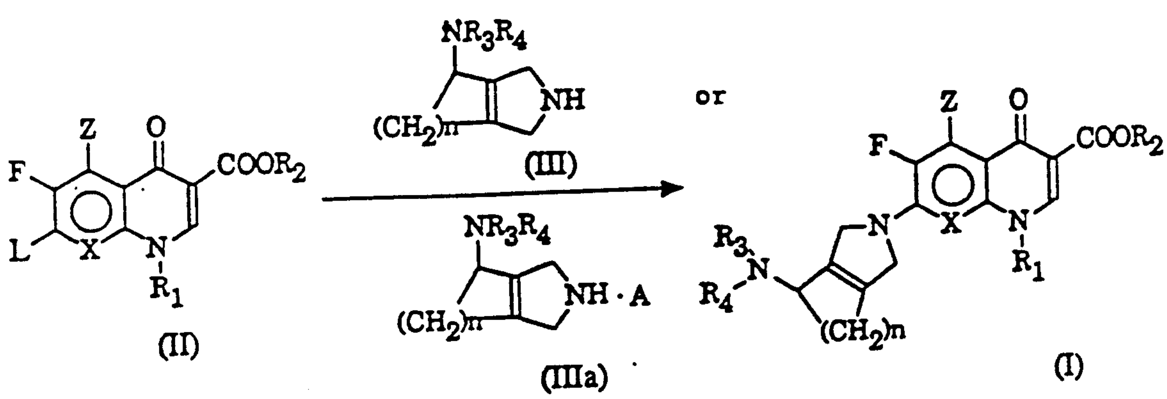

(II) (III) or (IIIa) (I)

wherein:

| | |
|---|---|
| $R_1$, $R_3$, $R_4$, X, Z and n are | the same as defined previously; |
| $R_2$ is | a hydrogen atom or a carboxy protecting group; |
| L is | a leaving group which is a fluorine, chlorine or bromine atom, a methanesulfonyl group or a para-toluenesulfonyl group; and, |
| A is | a hydrochloric, bromic, sulfuric or trifluoroacetic acid. |

In accordance with the above Process A, the quinolone compounds of formula(I) wherein $R_2$ is a hydrogen atom or a carboxy protecting group(hereinafter referred to as the compounds of formula (Ia)) can be prepared by reacting the compounds of formula(II) with the compounds of formula(III) or (IIIa) in the presence of a base.

The compounds of formula(II) are well-known in the art and the compounds of formula(III) are novel and can be prepared in accordance with the methods described in Korean Patent Application No. 91-18097 entitled "Unsaturated Azabicycloamine Compounds and Processes for the Preparation Thereof" (corr. to WO-A-...........).

The above reaction can be carried out in the presence of an inert solvent such as methanol, ethanol, isopropanol, acetonitrile, pyridine, dimethylformamide, dimethylsulfoxide, dioxane, etc. or a mixture thereof at a temperature ranging from 10 and 200 °C preferably 50 and 120 °C for 10 min to 24 hrs.

A base such as triethylamine, diisopropylethylamine, pyridine, 1,8-diazabicyclo[5,4,0]undec-7-ene or alkaline metal or alkaline earth metal carbonate may be used to neutralize the acids produced in the above reaction.

The compounds of formula(Ia) obtained from Process A wherein $R_2$ is a hydrogen atom(hereinafter referred to as the compounds of formula(Ia-1)) can be converted to the compounds of formula(Ia) wherein $R_2$ is a carboxy protecting group(hereinafter referred to as the compounds of formula(Ia-2)) in accordance with the following Process B.

Process B

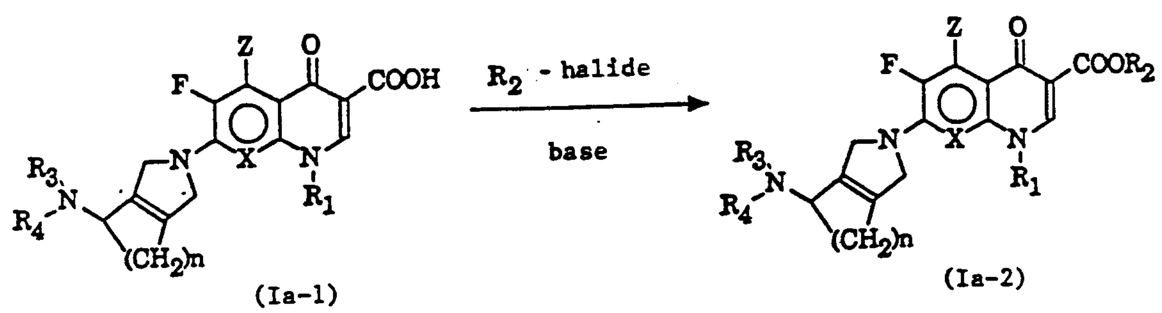


(Ia-1) (Ia-2)

wherein:

| | |
|---|---|
| $R_1$, $R_3$, $R_4$, Z, X and n are | the same as defined previously; |
| Hal is | a chlorine, bromine or iodine atom; and |
| $R_2$ is | a carboxy protecting group which is a lower alkyl group such as methyl, ethyl, n-propyl or t-butyl group, a lower alkanoyloxy-lower alkyl group such as acetoxymethyl or pivaloyloxymethyl group, a lower alkoxycarbonyloxy-lower alkyl group such as methoxycarbonyloxymethyl or 1-ethoxycarbonyloxyethyl group, a lower alkoxymethyl group such as methoxymethyl group, a di(lower alkyl) amino-lower alkyl group such a dimethylaminoethyl group or a 4-methylene-5-methyl-1,3-dioxolene-2-one group. |

In accordancce with the above Process B, the compounds of formula(Ia-2) can be prepared by reacting the compounds of formula (Ia-1) with the compounds of $R_2$-Hal in an inert solvent such as haloalkane or dimethylformamide in the presence of a base such as diisopropylethylamine.

The compounds of formula(Ia-1) obtained from Process A can be converted to the compounds of formula(Ia) wherein $R_2$ is a pharmaceutically acceptable metal or organic cation(hereinafter referred to as the compounds of formula(Ia-3)) in accordance with the following Process C.

Process C

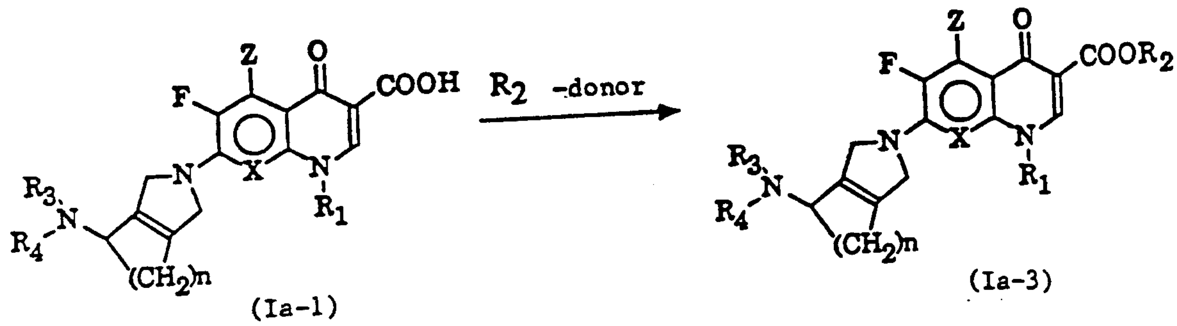


(Ia-1)

(Ia-3)

wherein:

$R_1$, $R_3$, $R_4$, Z, X and n are the same as defined previously; and
$R_2$ is a pharmaceutically acceptable metal or organic cation.

In accordance with the above Process C, the compounds of formula(Ia-3) can be prepared by dissolving the compounds of formula (Ia-1) in a lower alcohol or haloalkane solvent; adding thereto an $R_2$-donor in water or a lower alcohol; and collecting the produced precipitates or removing the solvent.

Said $R_2$-donor used in the above reaction may be sodium hydroxide, potassium hydroxide, calcium hydroxide, magnesium hydroxide, silver nitrate or organic cation compounds.

The compounds of formula(Ia-3) may further encompass, their hydrates.

The compounds of formula(Ia) obtained from Process A wherein $R_4$ is a hydrogen atom(hereinafter referred to as the compounds of formula(Ia-4)) can be converted to the compounds of formula(Ia) wherein $R_4$ is the same as defined in the formula(Ia), excepting a hydrogen atom(hereinafter referred to as the compounds of formula(Ia-5)) by using the following Process D.

Process D

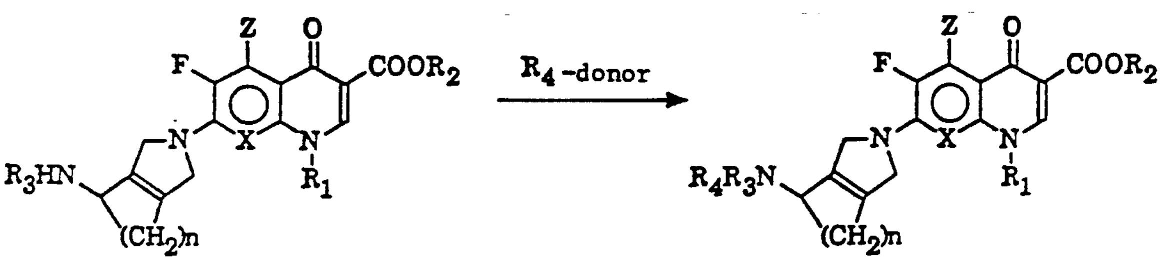


(Ia-4)

(Ia-5)

wherein:

$R_1$, $R_2$, $R_3$, Z, X and n are the same as defined in the formula (Ia); and
$R_4$ is the same as defined in the formula(Ia), except a hydrogen atom.

In accordance with the above Process D, the compounds of formula(Ia-5) can be prepared by reacting the compounds of formula (Ia-4) with an $R_4$-donor of formula $R_4$-Hal(wherein Hal is a chlorine, bromine or iodine atom) in the presence of a base such as triethylamine, diisopropylethylamine, 1,8-diazabicyclo[5,4,0]-undec-7-ene, sodium hydride, potassium hydride, calcium hydride or alkaline metal carbonate.

The above process is advantageous especially when $R_3$ is a hydrogen atom or an alkyl group and $R_4$ is a nitrogen protecting group metabolizable in vivo.

If $R_4$ is a formyl group, the compound of formula(Ia-4) is reacted with acetic anhydride or sodium acetate in formic acid; and if $R_4$ is an acyl group, with an acid anhydride or halide having two to four carbon atoms in the presence of a base such as triethylamine or pyridine.

The compounds of formula(Ia) may be converted to acid addition salts thereof with an inorganic or organic acid such as hydrochloric, sulfuric, phosphoric, nitric, methanesulfonic, acetic, formic, propionic,

lactic, maleic, malonic, fumaric, tartaric, citric, ascorbic, glutamic, paratoluenesulfonic acid, etc; and to their hydrates.

The above conversion reaction is carried out by dissolving the compounds of formula(Ia) in a solvent, e.g., a lower alcohol such as methanol or ethanol, or a haloalkane such as chloroform, dichloromethane or dichloroethane; adding an aqueous or alcoholic solution of a selected organic or inorganic acid; and collecting the precipitates produced therefrom or removing the solvent.

The present invention further encompasses a pharmaceutical composition comprising one or more compounds of formula(I) and non-toxic inert excipients.

The compounds of the present invention can be administered locally, orally, parenterally or rectally, preferably intravenously or intramuscularly. In general, it has been shown advantageous to administer the compounds of the present invention in an amout of about 0.5 to 500, preferably 1 to 100mg/kg of body weight per day in single or divided doses. A dosage ranging from about 1 to 200mg/kg of body weight, particularly 1 to 50mg/kg of body weight is preferred. However, it will be understood that the amount of the compound actually administered may be adjusted in the light of the relevant circumstances including the form of formulation, the chosen route of administration, the age, weight ad response of the individual patient, and the severity of the patient's symptoms.

One or more compounds of the present invention may be either administered as such or formulated for administration by mixing therewith non-toxic, inert pharmaceutically acceptable expients such as solid, semi-solid or liquid diluent, filler and carrier. Examples of such formulation are in the form of: a tablet, lozenge, capsule, granule, suppository, solution, suspension, emulsion, paste, ointment, cream, lotion, powder, spray and the like.

In case of tablet, lozenge, capsule and granule, the active compounds of the present invention may be combined with conventional expients, e.g., fillers and extenders such as starch, lactose, sucrose, glucose mannitol and the like, binders such as carboxymethyl cellulose, alginate, gelatine, polyvinylpyrolidone and the like; disintegrants such as calcium carbonate, sodium bicarbonate and the like; absorption accelerants such as quarternary ammonium compound and the like; wetting agents such as cetyl alcohol, glycerine monostearate and the like; adsorbents such as kaoline, bentonite and the like; lubricants such as talc, calcium stearate, magnesium stearate, solid polyethylene glycol and the like; or mixtures thereof. The tablet, lozenge, capsule, pill and granule may be coated with conventional coating materials including any opacifier.

The suppository may contain conventional soluble or insoluble expients, e.g., polyethylene glycol, fat, polymeric ester or a mixture thereof in addition to the active compound.

The ointment, paste, cream and the like may contain conventional expients, e.g., animal or vegetable fat, wax, paraffin, starch, cellulose derivatives, polyethylene glycol, bentonite, talc, zine oxide or a mixture thereof in addition to the active compounds.

The solution or emulsion for oral administration may contain conventional expients such as solvents, solubilizers and emulsifiers, e.g., water, ethyl alcohol, benzyl benzoate, propylene glycol, cotton seed oil, pinutts oil, corn oil, olive oil, fatty esters such as glycerine, polyethylene glycol or sorbitan, or a mixture thereof in addition to the active compounds.

The solution, suspension or emulsion for parenteral administration may contain a sterilized isometric solution or emulsion. In particular, the suspension may contain the expients, e.g., liquid diluent or suspending agent such as water, ethyl alcohol or propylene glycol.

The formulations may comprise about 0.1 to 99. 5% by weight, preferably 0.5 to 95% by weight of the active compounds of the present invention; and may further contain conventionally accepted amounts of dyes, preservatives, sweetners and additives.

It will be apparent to those skilled in the art that certain changes and modifications may be made to this invention without departing from the spirit or scope of the invention as it is further illustrated below.

Example 1: Preparation of 7-[6-amino-3-azabicyclo[3,3,0]oct-1(5) ene-3-yl]-1-cyclopropyl-6,8-difluoro-1,4-dihydro-4-oxoquinoline-3-carboxylic acid

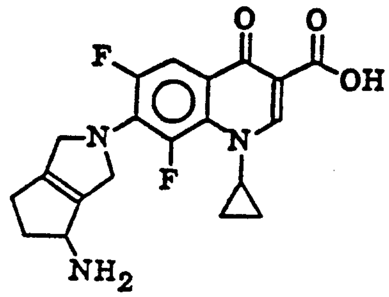

To 10mℓ of acetonitrile was added 330mg of 6-amino-3-azabicyclo[3,3,0]oct-1(5)ene dihydrobromide and 320mg of 1-cyclopropyl-6,7,8-trifluoro-1,4-dihydro-4-oxoquinoline-3-carboxylic acid; and the resulting solution was refluxed for 5 hours after an addition of 616μℓ of 1,8-diazabicyclo[5,4,0]undec-7-ene. The reaction mixture was cooled and filtered; and the residue was washed with acetonitrile to obtain 340mg of the desired compound in light yellow precipitate(yield: 85%).
m.p.: 213~215°C

| | |
|---|---|
| MS m/z (rel. int, %) : | 387 ($M^+$), 370 (100), 343 (27), 326 (73), 299 (25) |
| $^1$H-NMR ($CDCl_3$ + $CD_3COOD$, δ ppm) ; | 8.75 (1H, s), 7.76 (1H, dd, J = 14.7, 1.5 Hz), 4.63 (4H, br, s), 4.39 (1H, m), 4.1 (1H, m), 4.39 (1H, m), 4.1 (1H, m), 2.90 (1H, m), 2.61 (1H, m), 2.52 (1H, m), 2.35 (1H, m), 1.26 (4H, m) |

Example 2: Preparation of 1-cyclopropyl-6,8-difluoro-7-[6-methylamino-3-azabicyclo[3,3,0]oct-1(5)ene-3-yl]-1,4-dihydro-4-oxoquinoline-3-carboxylic acid

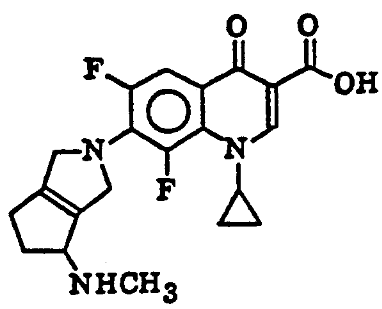

To 3mℓ of acetonitrile was added 40mg of 6-methylamino-3-azabicyclo[3,3,0]oct-1(5)ene dihydrobromide and 37.5mg of 1-cyclopropyl-6,7,8-trifluoro-1,4-dihydro-4-oxoquinoline-3-carboxylic acid; and the resulting solution was refluxed for 5 hours after an addition of 72μℓ of 1,8-diazabicyclo[5,4,0]undec-7-ene. The reaction mixture was cooled and filtered; and the residue was washed with acetonitrile to obtain 34mg of the desired compound in white precipitate(yield: 65%).
m.p.: 194~196°C

| | |
|---|---|
| MS m/z (rel. int, %) : | 401 ($M^+$), 370 (89), 326 (100), 299 (15), 285 (10), 258 (10), 237 (10), 220 (20) |
| $^1$H-NMR ($CDCl_3$ + $CD_3COOD$, δ ppm) ; | 8.74 (1H, s), 7.73 (1H, d, J = 14 Hz), 4.67 (4H, br, m), 4.39 (1H, br, s), 4.09 (1H, m), 2.85 (1H, m), 2.71 93H, s), 2.61 (1H, m), 2.50 (1H, m), 2.42 (1H, m), 1.21 (4H, m) |

Example 3: Preparation of 7-[6-amino-3-azabicyclo[3,3,0]oct-1(5) ene-3-yl]-1-cyclopropyl-8-chloro-6-fluoro-1,4-dihydro-4-oxoquinoline-3-carboxylic acid

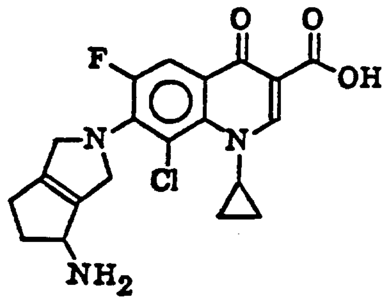

To 25mℓ of acetonitrile was added 800mg of 6-amino-3-azabicyclo[3,3,0]oct-1(5)ene dihydrobromide and 790mg of 1-cyclopropyl-6,7-difluoro-8-chloro-1,4-dihydro-4-oxoquinoline-3-carboxylic acid; and the resulting solution was refluxed for 5 hours after an addition of 1.38mℓ of 1,8-diazabicyclo[5,4,0]undec-7-ene. The reaction mixture was cooled and filtered; and the residue was washed with acetonitrile to obtain 400mg of the desired compound in white color(yield: 38%).
m.p.: 193~194 °C

| | |
|---|---|
| MS m/z (rel. int, %): | 403 ($M^+$, 10), 386 (72), 359 (27), 342 (100), 307 (62), 282 (65), 252 (51), 216 (47), 201 (32), 172 (27), 108 (35) |
| $^1H$-NMR ($CDCl_3$ + $CD_3COOD$, δ ppm) ; | 8.97 (1H, s), 8.03 (1H, d, J = 12 Hz), 4.46~4.34 (6H, m), 2.80 (1H, br, s), 2.61 (1H, br, s), 2.46 (2H, br, s), 1.32 (2H, m), 0.98 (2H, m) |

Example 4: Preparation of 7-[6-methylamino-3-azabicyclo[3,3,0]oct-1(5)ene-3-yl]-8-chloro-1-cyclopropyl-6-fluoro-1,4-dihydro-4-oxoquinoline-3-carboxylic acid

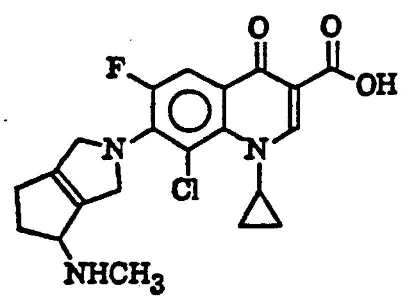

To 2.5mℓ of acetonitrile was added 100mg of 6-methylamino-3-azabicyclo[3,3,0]oct-1(5)ene dihydrobromide and 95mg of 1-cyclopropyl-6,7-difluoro-1,4-dihydro-4-oxoquinoline-3-carboxylic acid; and the resulting solution was refluxed for 4 hours after an addition of 164µℓ of 1,8-diazabicyclo[5,4,0]undec-7-ene. The reaction mixture was cooled and filtered; and the residue was washed with acetonitrile to obtain 80mg of the desired compound in white color(yield: 60%).
m.p.: 207~208 °C

| | |
|---|---|
| MS m/z (rel. int, %): | 417 ($M^+$, 6), 386 (90), 342 (100), 308 (46), 288 (29), 263 (22), 216 (27), 122 (50), 109 (68) |
| $^1H$-NMR ($CDCl_3$ + $CD_3COOD$, δ ppm) ; | 8.95 (1H, s), 8.04 (1H, d, J = 14.4 Hz), 4.46~4.33 (6H, br, m), 2.7 (3H, s), 2.82~2.49 (4H, m), 1.32 (2H, m), 0.98 (2H, m) |

Example 5: Preparation of 7-[6-amino-3-azabicyclo[3,3,0]oct-1(5) ene-3-yl]-8-bromo-1-cyclopropyl-6-fluoro-1,4-dihydro-4-oxoquinoline-3-carboxylic acid

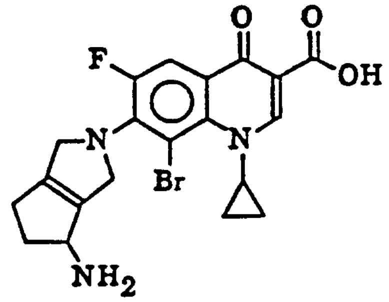

320mg of 6-amino-3-azabicyclo[3,3,0]oct-1(5)ene dihydrobromide and 344mg of 8-bromo-1-cyclopropyl-6,7-difluoro-1,4-dihydro-4-oxoquinoline-3-carboxylic acid were subjected to the same process as described in Example 1 to obtain 271mg of the desired compound (yield: 70%).
m.p.: 209~213 °C

$^1$H-NMR ($CDCl_3$ + $CD_3COOD$, δ ppm) ; 8.15 (1H, s), 7.49 (1H, d, J=12.0 Hz), 4.48 (4H, br. s), 4.35 (1H, m), 4.01 (1H, m), 2.92 (1H, m), 2.63 (1H, m), 2.50 (1H, m), 2.34 (1H, m), 1.20-1.05 (4H, m)

Example 6: Preparation of 7-[6-amino-3-azabicyclo[3,3,0]oct-1(5) ene-3-yl]-1-cyclopropyl-6-fluoro-8-methoxy-1,4-dihydro-4-oxoquinoline-3-carboxylic acid

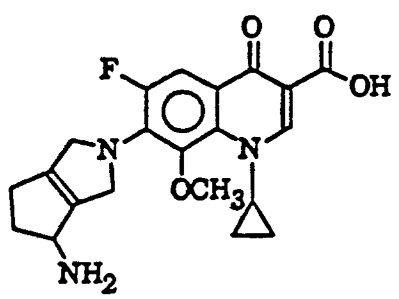

320mg of 6-amino-3-azabicyclo[3,3,0]oct-1(5)ene dihydrobromide and 295mg of 1-cyclopropyl-6,7-difluoro-8-methoxy-1,4-dihydro-4-oxoquinoline-3-carboxylic acid were subjected to the same process as described in Example 1 to obtain 232mg of the desired compound (yield: 58%).
m.p.: 195~198 °C

$^1$H-NMR ($CDCl_3$ + $CD_3COOD$, δ ppm) ; 8.84 (1H, s), 7.69 (1H, d, J = 13.8 Hz), 4.55~4.35 (5H, br, s), 4.05 (1H, m), 3.59 (3H, s), 2.95~2.30 (4H, m), 1.23~1.05 (4H, m)

Example 7: Preparation of 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxylic acid

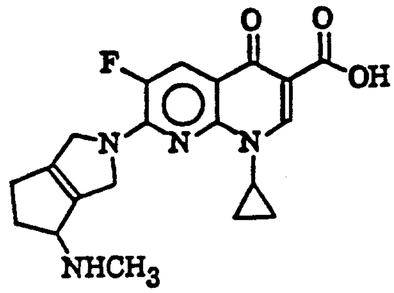

To 2mℓ of acetonitrile was added 70mg of 6-methylamino-3-azabicyclo[3,3,0]oct-1(5)ene dihydrobromide and 62mg of 1-cyclopropyl-7-chloro-6-fluoro-4-oxo-1,8-naphthyridine-3-carboxylic acid; and the resulting solution was refluxed for 4 hours after an addition of 62mg of 1,8-diazabicyclo[5,4,0]undec-7-ene. The reaction mixture was cooled, concentrated under a reduced pressure and fractionated on silica gel column chromatography(eluent:chloroform/methanol/water = 15/3/1) to obtain 60mg of the desired compound in yellow color (yield: 71%).
m.p.: 185~190 °C

| | |
|---|---|
| MS m/z (rel. int, %): | 384 ($M^+$, 8), 353 (17), 342 (100), 311 (62), 296 (55), 284 (15), 218 (15), 163 (10), 122 (15), 109 (15) |
| $^1H$-NMR ($CDCl_3$ + $CD_3COOD$, δ ppm) ; | 8.69 (1H, s), 7.98 (1H, br, s), 4.63~4.45 (5H, br, m), 3.64 (1H, br, s), 2.68 (3H, s), 2.8~2.45 (4H, m), 1.28 (2H, br, s), 1.07 (2H, br, s) |

Example 8: Preparation of 7-[6-amino-3-azabicyclo[3,3,0]oct-1(5) ene-3-yl]-6,8-difluoro-1-ethyl-1,4-dihydro-4-oxoquinoline-3-carboxylic acid

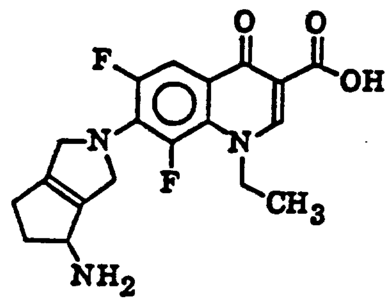

320mg of 6-amino-3-azabicyclo[3,3,0]oct-1(5)ene dihydrobromide and 343mg of 1-ethyl-6,7,8-trifluoro-1,4-dihydro-4-oxoquinoline-3-carboxylic acid were subjected to the same process as described in Example 1 to obtain 291mg of the desired compound (yield: 65%).
m.p.: 245~249 °C (decomposition)

| | |
|---|---|
| $^1H$-NMR ($CDCl_3$ + $CD_3COOD$, δ ppm) ; | 8.78 (1H, s), 7.86 (1H, d, J = 14.2 Hz), 4.60 (4H, br. s), 4.31 (1H, m), 4.23 (2H, q, J = 7 Hz), 2.94 (1H, m), 2.64 (1H, m), 2.51 (1H, m), 2.35 (1H, m), 1.45 (3H, t, J = 7 Hz) |

Example 9: Preparation of 7-[6-amino-3-azabicyclo[4,3,0]non-1(5) ene-3-yl]-1-cyclopropyl-6,8-difluoro-1,4-dihydro-4-oxoquinoline-3-carboxylic acid

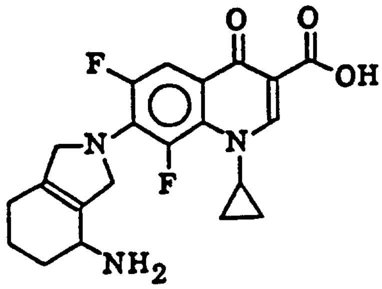

To 10mℓ of acetonitrile was added 400mg of 6-amino-3-azabicyclo[4,3,0]non-1(5)ene dihydrobromide and 365mg of 1-cyclopropyl-6,7,8-trifluoro-4-oxoquinoline-3-carboxylic acid; and the resulting solution was refluxed for 5 hours after a addition of 735μℓ of 1,8-diazabicyclo[5,4,0]undec-7-ene. The reaction mixture was cooled and filtered; and the residue was washed with acetonitrile to obtain 387mg of the desired compound in light yellow precipitate (yield: 75%).
m.p,: 200~204°C

| | |
|---|---|
| $^1$H-NMR ($CDCl_3$ + $CD_3COOD$, δ ppm) ; | 8.76 (1H, s), 7.73 (1H, d, J=13Hz), 4.68 (1H, br, s), 4.64 (4H, br, s), 4.42 (1H, br, s), 4.10 (1H, br, s), 2.91 (1H, m), 2.65 (1H, m), 2.55 (1H, m), 2.35 (1H, m), 1.47 (2H, m), 1.25 (4H, m) |

Example 10: Preparation of 7-[6-amino-3-azabicyclo[3,3,0]oct-1(5) ene-3-yl]-5-amino-1-cyclopropyl-6,8-difluoro-1,4-dihydro-4-oxoquinoline-3-carboxylic acid

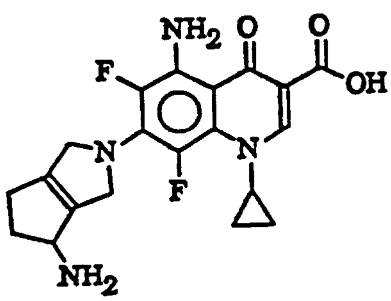

To 2mℓ of acetonitrile was added 50mg of 6-amino-3-azabicyclo[3,3,0]oct-1(5)ene dihydrobromide and 50mg of 5-amino-1-cyclopropyl-6,7,8-trifluoro-1,4-dihydro-4-oxoquinoline-3-carboxylic acid; and the resulting solution was refluxed for 5 hours after an addition of 86μℓ of 1,8-diazabicyclo[5,4,0]undec-7-ene. The reaction mixture was cooled and filtered; and the residue was washed with acetonitrile to obtain 35mg of the desired compound in light yellow color(yield: 50%).
m.p.: 134~137°C

| | |
|---|---|
| MS m/z (rel. int, %) : | 407 ($M^+$, 23), 385 (14), 358 (45), 295 (23), 231 (15), 50 (100), 137 (40) |
| $^1$H-NMR ($CDCl_3$ + $CD_3COOD$, δ ppm) ; | 8.63 (1H, s), 4.55 (5H, br, s), 3.91 (1H, br, s), 2.78~2.43 (4H, br, m), 1.18 (2H, br, s), 1.04 (2H, br, s) |

Example 11: Preparation of 7-[6-methylamino-3-azabicyclo[3,3,0]oct-1(5)ene-3-yl]-5-amino-1-cyclopropyl-6,8-difluoro-1,4-dihydro-4-oxoquinoline-3-carboxylic acid

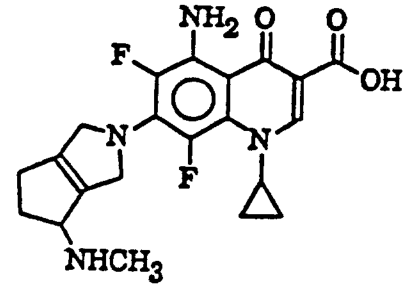

To 2mℓ of acetonitrile was added 70mg of 6-methylamino-3-azabicyclo[3,3,0]oct-1(5)ene dihydrobromide and 66mg of 5-amino-1-cyclopropyl-6,7,8-trifluoro-1,4-dihydro-4-oxoquinoline-3-carboxylic acid; and the resulting solution was refluxed for 4 hours after an addition of 115μℓ of 1,8-diazabicyclo-[5,4,0]undec-7-ene. The reaction mixture was cooled and filtered; and the residue was washed with acetonitrile to obtain 73mg of the desired compound in light yellow color(yield: 80%).
m.p.: 222~224°C

| | |
|---|---|
| MS m/z (rel. int, %): | 416 ($M^+$, 95), 385 (100), 372 (37), 320 (38), 300 (45), 231 (87), 216 (25), 122 (56), 109 (57) |
| $^1$H-NMR ($CDCl_3$ + $CD_3COOD$, δ ppm) ; | 8.63 (1H, s), 4.65~4.41 (5H, m), 3.90 (1H, br, s), 2.68 (3H, s), 2.80~2.44 (4H, m), 1.19 (2H, m), 1.05 (2H, m) |

Example 12: Preparation of 7-[6-amino-3-azabicyclo[3,3,0]oct-1(5) ene-3-yl]-1-(2,4-difluorophenyl)-6,8-difluoro-1,4-dihydro-4-oxoquinoline-3-carboxylic acid

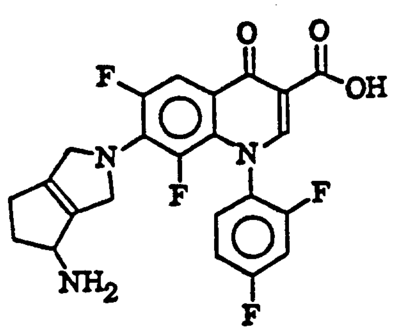

To 1.2mℓ of acetonitrile was added 65mg of 6-amino-3-azabicyclo[3,3,0]oct-1(5)ene dihydrobromide and 70mg of 1-(2,4-difluorophenyl)-6,7,8-trifluoro-1,4-dihydro-4-oxoquinoline-3-carboxylic acid; and the resulting solution was refluxed for 4 hours after an addition of 91μℓ of 1,8-diazabicyclo[5,4,0]undec-7-ene. The reaction mixture was cooled and filtered; and the residue was washed with acetonitrile and ethylether to obtain 45mg of the desired compound(yield: 50%).
m.p: 185~190°C (decomposition)

| | |
|---|---|
| MS m/z (rel. int, %): | 459 ($M^+$, 1), 442 (3), 415 (30), 398 (100), 371 (40), 321 (15), 292 (17), 151 (10), 123 (14) |
| $^1$H-NMR ($CDCl_3$ + $CD_3COOD$, δ ppm) ; | 8.49 (1H, s), 7.91 (1H, d, J = 14 Hz), 8.17 (1H, m), 7.08 (2H, m), 4.50~4.30 (5H, m), 2.71~2.32 (4H, m) |

Example 13: Preparation of 7-[6-methylamino-3-azabicyclo[3,3,0]oct-1(5)ene-3-yl]-1-(2,4-difluorophenyl)-6,8-difluoro-1,4-dihydro-4-oxoquinoline-3-carboxylic acid

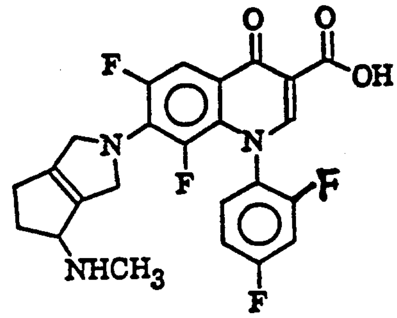

To 1.2mℓ of acetonitrile was added 65mg of 6-methylamino-3-azabicyclo[3,3,0]oct-1(5)ene dihydrobromide and 70mg of 1-(2,4-difluorophenyl)-6,7,8-trifluoro-1,4-dihydro-4-oxoquinoline-3-carboxylic acid; and the resulting solution was refluxed for 4 hours after an addition of 91μℓ of 1,8-diazabicyclo[5,4,0]-undec-7-ene. The reaction mixture was cooled and filtered; and the residue was washed with acetonitrile and ethylether to obtain 60mg of the desired compound(yield: 65%).
m.p.: 228~300 °C

| | |
|---|---|
| MS m/z (rel. int, %): | 473 ($M^+$, 3), 441 (13), 430 (25), 399 (100), 372 (23), 307 (10, 237 (6), 122 (10), 109 (15), 94 (10) |
| $^1$H-NMR ($CDCl_3$ + $CD_3COOD$, δ ppm) ; | 8.48 (1H, s), 7.89 (1H, dd, J = 13, 1.5 Hz), 7.50 (1H, m), 7.08 (2H, m), 4.56~4.31 (5H, m), 2.74~2.39 (4H, m), 2.62 (3H, s) |

Example 14: Preparation of 7-[6-amino-3-azabicyclo[3,3,0]oct-1(5) ene-3-yl]-1-cyclopropyl-6-fluoro-1,4-dihydro-4-oxo-1,8-naphthyridine-3-carboxylic acid

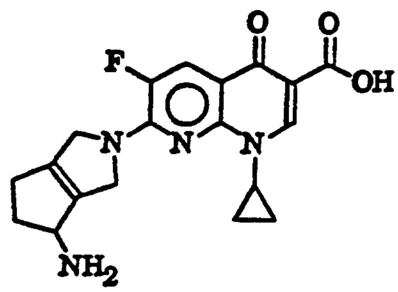

320mg of 6-amino-3-azabicyclo[3,3,0]oct-1(5)ene dihydrobromide and 282mg of 1-cyclopropyl-7-chloro-6-fluoro-4-oxo-1,8-naphthyridine-3-carboxylic acid were subjected to the same process as described in Example 1 to obtain 229mg of the desired compound (yield: 59%).
m.p.: 175~179 °C

| | |
|---|---|
| $^1$H-NMR ($CDCl_3$ + $CD_3COOD$, δ ppm) ; | 8.72 (1H, s), 7.82 (1H, br, s), 4.71~4.52 (5H, br, m), 3.61 (1H, br, s), 2.8~2.39 (4H, m), 1.28 (2H, br, s), 1.05 (2H, br, s) |

Example 15: Preparation of 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxylic acid

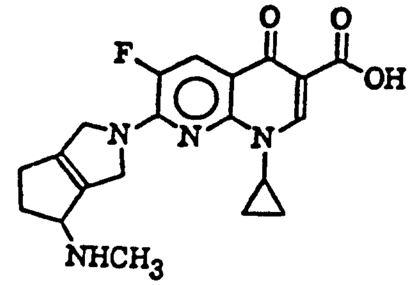

To 2mℓ of acetonitrile was added 70mg of 6-methylamino-3-azabicyclo[3,3,0]oct-1(5)ene dihydrobromide and 62mg of 1-cyclopropyl-7-chloro-6-fluoro-4-oxo-1,8-naphthyridine-3-carboxylic acid; and the resulting solution was refluxed for 4 hours after an addition of 62mg of 1,8-diazabicyclo[5,4,0]undece-7-ene. The reaction mixture was cooled, concentrated under a reduced pressure and fractionated on silica gel column chromatography(eluent: chloroform/methanol/water = 15/3/1) to obtain 60mg of the desired compound in yellow color(yield: 71%).
m.p.: 185~190 °C

| | |
|---|---|
| MS m/z (rel. int, %) : | 384 ($M^+$, 8), 353 (17), 342 (100), 311 (62), 296 (55), 284 (15), 218 (15), 163 (10), 122 (15), 109 (15) |
| $^1$H-NMR ($CDCl_3$ + $CD_3COOD$, δ ppm) ; | 8.69 (1H, s), 7.98 (1H, br, s), 4.63~4.45 (5H, br, m), 3.64 (1H, br, s), 2.68 (3H, s), 2.8~2.45 (4H, m), 1.28 (2H, br, s), 1.07 (2H, br, s) |

Example 16: Preparation of 9-fluoro-10-[6-amino-3-azabicyclo[3,3,0] oct-1(5)ene-3-yl]-3-(S)-methyl-7-oxo-2,3-dihydro-7H-pyrido[1,2,3-de]-1,4-benzoxazine-6-carboxylic acid

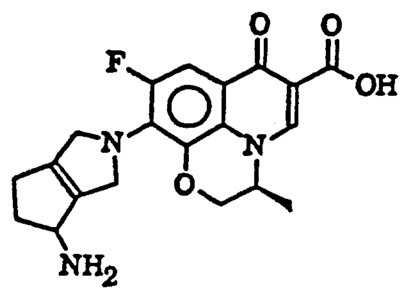

320mg of 6-amino-3-azabicyclo[3,3,0]oct-1(5)ene dihydrobromide and 295mg of 9,10-difluoro-3-(S)-methyl-7-oxo-2,3-dihydro-7H-pyrido[1,2,3-de]-1,4-benzoxazine-6-carboxylic acid were subjected to the same process as described in Example 1 to obtain 196mg of the desired compound(yield: 49%).
m.p.: 183~186 °C

| | |
|---|---|
| MS m/z (rel. int, %) : | 399 ($M^+$, 7), 368 (40), 357 (27), 326 (100), 234 (57), 219 (67), 205 (35), 193 (31), 122 (44), 109 (47) |
| $^1$H-NMR ($CDCl_3$ + $CD_3COOD$, δ ppm) ; | 8.75 (1H, s), 7.65 (1H, d, J = 14.2 Hz), 4.65~4.30 (6H, m), 4.25 (2H, br, s), 2.98~2.35 (4H, m), 1.67 (3H, d, J = 6.5 Hz) |

Example 17: Preparation of 9-fluoro-10-[6-methylamino-3-azabicyclo [3,3,0]oct-1(5)ene-3-yl]-3-(S)-methyl-7-oxo-2,3-dihydro-7H-pyrido[1,2,3-de]-1,4-benzoxazine-6-carboxylic acid

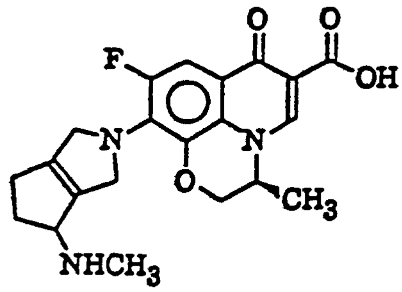

To 1.5mℓ of acetonitrile was added 70mg of 6-methylamino-3-azabicyclo[3,3,0]oct-1(5)ene dihydrobromide and 63mg of 9,10-difluoro-3-(S)-methyl-7-oxo-2,3-dihydro-7H-pyrido[1,2,3-de]-1,4-benzeoxazine-6-carboxylic acid; and the resulting solution was refluxed for 4 hours after an addition of 115μℓ of 1,8-diazabicyclo [5,4,0]undece-7-ene. The reaction mixture was cooled, filterted and washed with acetonitrile to obtain 45mg of the desired compound (yield: 50%).
m.p.: 186~188 °C

| | |
|---|---|
| MS m/z (rel. int, %) : | 399 ($M^+$, 7), 368 (40), 357 (27), 326 (100), 234 (57), 219 (67), 205 (35), 193 (31), 122 (44), 109 (47) |
| $^1$H-NMR ($CDCl_3$ + $CD_3COOD$, δ ppm) ; | 8.78 (1H, s), 7.68 (1H, dd, J = 13 Hz, 1.2 Hz), 4.67~4.28 (8H, m), 2.67 (3H, s), 2.80~2.47 (4H, m), 1.61 (3H, d, J = 6.7 Hz) |

Example 18: Preparation of 7-[6-amino-3-azabicyclo[3,3,0]oct-1(5) ene-3-yl]-1-ethyl-6-fluoro-1,4-dihydro-4-oxoquinoline-3-carboxylic acid

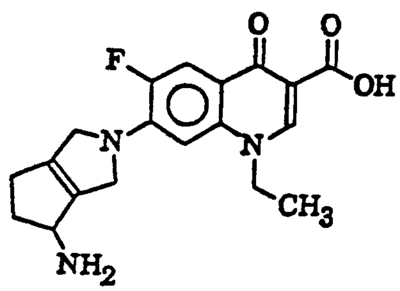

320mg of 6-amino-3-azabicyclo[3,3,0]oct-1(5)ene dihydrobromide and 252mg of 6,7-difluoro-1-ethyl-1,4-dihydro-4-oxoquinoline-3-carboxylic acid were subjected to the same process as described in Example 1 to obtain 260mg of the desired compound(yield: 73%).
m.p.: 212~215 °C

| | |
|---|---|
| $^1$H-NMR ($CDCl_3$ + $CD_3COOD$, δ ppm) ; | 8.75 (1H, s), 7.85 (1H, d, J = 12.4 Hz), 7.12 (1H, d, J = 7.3 Hz), 4.45 (4H, br, s), 4.31 (1H, m), 4.09 (2H, q, J = 7 Hz), 2.42 (2H, m), 2.18 (2H, m), 1.42 (3H, t, J = 7 Hz) |

Example 19: Preparation of 7-[6-methylamino-3-azabicyclo[3,3,0] oct-1(5)ene-3-yl]-1-ethyl-6-fluoro-1,4-dihydro-4-oxoquinoline-3-carboxylic acid

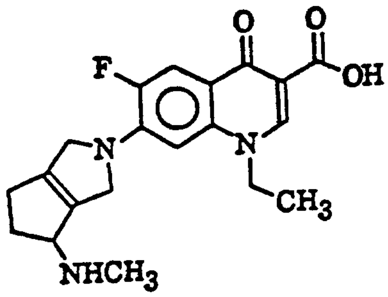

350mg of 6-methylamino-3-azabicyclo[3,3,0]oct-1(5)ene dihydrobromide and 252mg of 6,7-difluoro-1-ethyl-1,4-dihydro-4-oxoquinoline-3-carboxylic acid were subjected to the same process as described in Example 1 to obtain 241mg of the desired compound (yield: 65%).
m.p.: 220~224 °C

$^1$H-NMR ($CDCl_3$ + $CD_3COOD$, δ ppm) ; 8.69 (1H, s), 7.76 (1H, d, J = 12.4 Hz), 7.69 (1H, d, J = 7.3 Hz), 4.50~4.39 (5H, br, s), 4.05 (2H, q, J = 7 Hz), 2.91 (1H, m), 2.70 (3H, s), 2.49~2.20 (3H, br, s), 1.41 (3H, t, J = 7 Hz)

Example 20: Preparation of 7-[6-amino-3-azabicyclo[3,3,0]oct-1(5) ene-3-yl]-1-ethyl-6-fluoro-1,4-dihydro-4-oxoquinoline-3-carboxylic acid

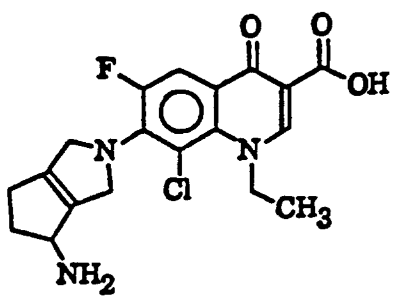

320mg of 6-amino-3-azabicyclo[3,3,0]oct-1(5)ene dihydrobromide and 286mg of 1-ethyl-6,7-difluoro-8-chloro-1,4-dihydro-4-oxoquinoline-3-carboxylic acid were subejcted to the same process as described in Example 1 to obtain 246mg of the desired compound (yield: 63%).
m.p.: 218~221 °C

$^1$H-NMR ($CDCl_3$ + $CD_3COOD$, δ ppm) ; 8.95 (1H, s), 7.98 (1H, d, J = 14.1 Hz), 4.58 (5H, br, s), 4.21 (2H, q, J = 7 Hz), 2.62 (1H, m), 2.51 (2H, m), 2.31 (1H, m), 1.44 (3H, t, J = 7Hz)

Example 21: Preparation of 7-[6-methylamino-3-azabicyclo[3,3,0]oct-1(5)ene-3-yl]-1-ethyl-6-fluoro-8-chloro-1,4-dihydro-4-oxoquinoline-3-carboxylic acid

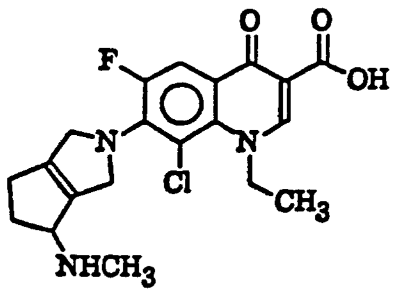

350mg of 6-methylamino-3-azabicyclo[3,3,0]oct-1(5)ene dihydrobromide and 286mg of 1-ethyl-6,7-difluoro-8-chloro-1,4-dihydro-4-oxoquinoline-3-carboxylic acid were subjected to the same process as described in Example 1 to obtain 223mg of the desired compound (yield: 55%).
m.p.: 223~227 ° C

$^1$H-NMR ($CDCl_3$ + $CD_3COOD$, δ ppm) ; 8.93 (1H, s), 7.89 (1H, d, J = 14.1 Hz), 4.59~4.39 (5H, br, s), 4.20 (2H, q, J = 7 Hz), 2.69 (3H, s), 2.60 (1H, m), 2.53 (2H, m), 2.30 (1H, m), 1.43 (3H, t, J = 7 Hz)

Example 22: Preparation of 7-[6-amino-3-azabicyclo[3,3,0]oct-1(5) ene-3-yl]-1-ethyl-5-amino-6,8-difluoro-1,4-dihydro-4-oxoquinoline-3-carboxylic acid

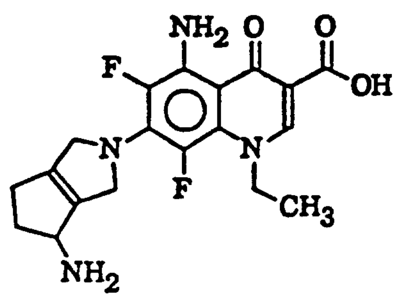

320mg of 6-amino-3-azabicyclo[3,3,0]oct-1(5)ene dihydrobromide and 286mg of 1-ethyl-5-amino-6,7,8-trifluoro-1,4-dihydro-4-oxoquinoline-3-carboxylic acid were subjected to the same process as described in Example 1 to obtain 242mg of the desired compound (yield: 62%).
m.p.: 238~243 ° C

$^1$H-NMR ($CDCl_3$ + $CD_3COOD$, δ ppm) ; 8.71 (1H, s), 4.52 (4H, br, s), 4.31 (1H, m), 4.21 (2H, t, J = 7 Hz), 2.92 (1H, m), 2.64 (1H, m), 2.48 (1H, m), 2.33 (1H, m), 1.42 (3H, t, J = 7 Hz)

Example 23: Preparation of 7-[6-methylamino-3-azabicyclo[3,3,0]oct-1(5)ene-3-yl]-1-ethyl-5-amino-6,8-difluoro-1,4-dihydro-4-oxoquinoline-3-carboxylic acid

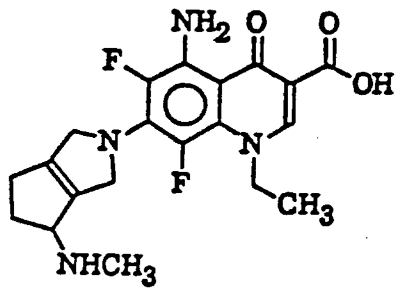

350mg of 6-methylamino-3-azabicyclo[3,3,0]oct-1(5)ene dihydrobromide and 286mg of 1-ethyl-5-amino-6,7,8-trifluoro-1,4-dihydro-4-oxoquinoline-3-carboxylic acid were subjected to the same process as described in Example 1 to obtain 223mg of the desired compound(yield: 55%).
m.p.: 218~221 ° C

$^1$H-NMR ($CDCl_3$ + $CD_3COOD$, δ ppm) ; 8.69 (1H, s), 4.50~4.41 (5H, br, s), 4.18 (2H, t, J = 7 Hz), 2.90 (1H, m), 2.70 (3H, s), 2.65 (1H, m), 2.47 (1H, m), 2.31 (1H, m), 1.38 (3H, t, J = 7 Hz)

Example 24: Preparation of 7-[6-amino-3-azabicyclo[3,3,0]oct-1(5) ene-3-yl]-1-(t-butyl)-6,8-difluoro-1,4-dihydro-4-oxoquinoline-3-carboxylic acid

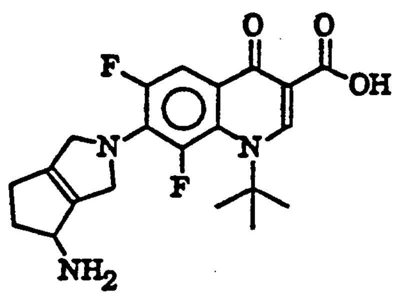

330mg of 6-amino-3-azabicyclo[3,3,0]oct-1(5)ene dihydrobromide and 299mg of 6,7,8-tirfluoro-1-(t-butyl)-1,4-dihydro-4-oxoquinoline-3-carboxylic acid were subjected to the same process as described in Example 1 to obtain 222mg of the desired compound (yield: 55%).
m.p.: 189~192 ° C

$^1$H-NMR ($CDCl_3$ + $CD_3COOD$, δ ppm) ; 8.58 (1H, s), 8.12 (1H, d, J = 14.2 Hz), 4.51~4.35 (5H, m), 2.91 (1H, m), 2.60~2.45 (2H, m), 2.31 (1H, m), 1.83 (9H, s)

Example 25: Preparation of 7-[6-methylamino-3-azabicyclo[3,3,0]oct-1(5)ene-3-yl]-1-(t-butyl)-6,8-difluoro-1,4-dihydro-4-oxoquinoline-3-carboxylic acid

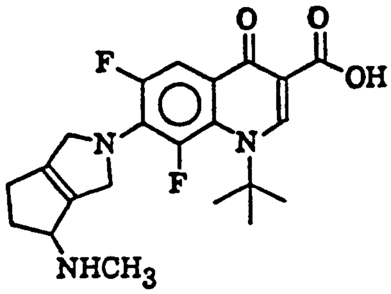

355mg of 6-methylamino-3-azabicyclo[3,3,0]oct-1(5)ene dihydrobromide and 299mg of 6,7,8-trifluoro-1-(t-butyl)-1,4-dihydro-4-oxoquinoline-3-carboxylic acid were subjected to the same process as described in Example 1 to obtain 238mg of the desired compound (yield: 57%).
m.p.: 205~208 °C

$^1$H-NMR ($CDCl_3$ + $CD_3COOD$, δ ppm) ; 8.56 (1H, s), 8.15 (1H, d, J = 14.2 Hz), 4.50~4.30 (5H, br, s), 2.93 (1H, m), 2.71 (3H, s), 2.62~2.43 (2H, m), 2.30 (1H, m), 1.81 (9H, s)

Example 26: Preparation of 7-[6-amino-3-azabicyclo[3,3,0]oct-1(5) ene-3-yl]-1-(t-butyl)-6-fluoro-8-chloro-1,4-dihydro-4-oxoquinoline-3-carboxylic acid

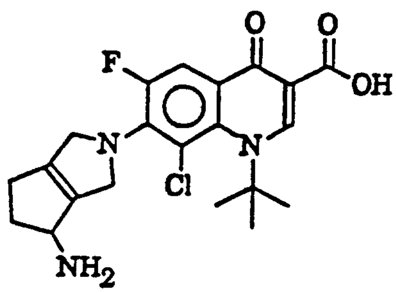

325mg of 6-amino-3-azabicyclo[3,3,0]oct-1(5)ene dihydrobromide and 315mg of 6,7-difluoro-8-chloro-1-(t-butyl)-1,4-dihydro-4-oxoquinoline-3-carboxylic acid were subjected to the same process as described in Example 1 to obtain 206mg of the desired compound (yield: 49%).
m.p.: 179~184 °C

$^1$H-NMR ($CDCl_3$ + $CD_3COOD$, δ ppm) ; 8.55 (1H, s), 8.09 (1H, d, J = 14.2 Hz), 4.51~4.32 (5H, m), 2.95 (1H, m), 2.65~2.42 (2H, m), 2.25 (1H, m), 1.85 (9H, s)

Example 27: Preparation of 7-[6-methylamino-3-azabicyclo[3,3,0]oct-1(5)ene-3-yl]-1-(t-butyl)-6-fluoro-8-chloro-1,4-dihydro-4-oxoquinoline-3-carboxylic acid

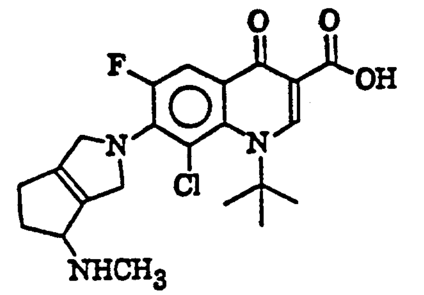

355mg of 6-methylamino-3-azabicyclo[3,3,0]oct-1(5)ene dihydrobromide and 315mg of 6,7-difluoro-8-chloro-1-(t-butyl)-1,4-dihydro-4-oxoquinoline-3-carboxylic acid were subejcted to the same process as described in Example 1 to obtain 195mg of the desired compound(yield: 45%).
m.p.: 175~178 °C

$^1$H-NMR ($CDCl_3$ + $CD_3COOD$, δ ppm) ; 8.54 (1H, s), 8.05 (1H, d, J = 14.2 Hz), 4.55~4.30 (5H, m), 2.95 (1H, m), 2.72 (3H, s), 2.65~2.45 (2H, m), 2.24 (1H, m), 1.84 (9H, s)

Example 28: Preparation of 7-[6-amino-3-azabicyclo[3,3,0]oct-1(5) ene-3-yl]-1-(4-fluorophenyl)-6,8-difluoro-1,4-dihydro-4-oxoquinoline-3-carboxylic acid

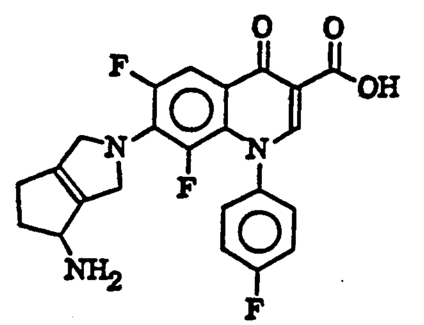

325mg of 6-amino-3-azabicyclo[3,3,0]oct-1(5)ene dihydrobromide and 337mg of 1-(4-fluorophenyl)-6,7,8-trifluoro-1,4-dihydro-4-oxoquinoline-3-carboxylic acid were subjected to the same process as described in Example 1 to obtain 274mg of the desired compound (yield: 62%).
m.p.: 221~224 °C

$^1$H-NMR ($CDCl_3$ + $CD_3COOD$, δ ppm) ; 8.59 (1H, s), 8.01 (1H, d, J = 14.2 Hz), 7.49 (2H, m), 7.25 (2H, m), 4.75~4.49 (5H, m), 2.91~2.37 (4H, m)

Example 29: Preparation of 7-[6-methylamino-3-azabicyclo[3,3,0]oct-1(5)ene-3-yl]-1-(4-fluorophenyl)-6,8-difluoro-1,4-dihydro-4-oxoquinoline-3-carboxylic acid

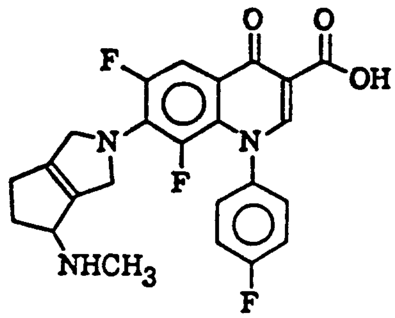

355mg of 6-methylamino-3-azabicyclo[3,3,0]oct-1(5)ene dihydrobromide and 337mg of 1-(4-fluorophenyl)-6,7,8-trifluoro-1,4-dihydro-4-oxoquinoline-3-carboxylic acid were subejcted to the same process as described in Example 1 to obtain 296mg of the desired compound(yield: 65%).
m.p.: 227~230 °C

$^1$H-NMR ($CDCl_3$ + $CD_3COOD$, $\delta$ ppm) ; 8.57 (1H, s), 7.99 (1H, d, J = 14.2 Hz), 7.48 (2H, m), 7.26 (2H, m), 4.76~4.51 (5H, m), 2.73 (3H, s), 2.90~2.36 (4H, m)

Example 30: Preparation of 7-[6-amino-3-azabicyclo[3,3,0]oct-1(5) ene-3-yl]-1-ethyl-6-fluoro-1,4-dihydro-4-oxo-1,8-naphthyridine-3-carboxylic acid

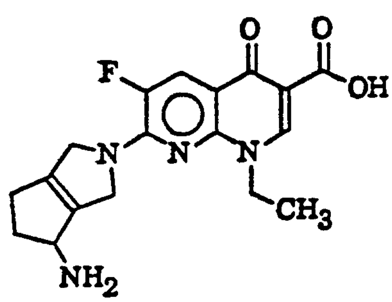

325mg of 6-amino-3-azabicyclo[3,3,0]oct-1(5)ene dihydrobromide and 270mg of 1-ethyl-7-chloro-6-fluoro-4-oxo-1,8-naphthyridine-3-carboxylic acid were subjected to the same process as described in Example 1 to obtain 205mg of the desired compound (yield: 57%).
m.p: 179~183 °C

$^1$H-NMR ($CDCl_3$ + $CD_3COOD$, $\delta$ ppm) ; 8.75 (1H, s), 8.09 (1H, d, J = 12.4 Hz), 4.75~4.35 (7H, m), 2.90~2.45 (4H, m), 1.51 (3H, t, J = 7 Hz)

Example 31: Preparation of 7-[6-methylamino-3-azabicyclo[3,3,0]oct-1(5)ene-3-yl]-1-ethyl-6-fluoro-1,4-dihydro-4-oxo-1,8-naphthyridine-3-carboxylic acid

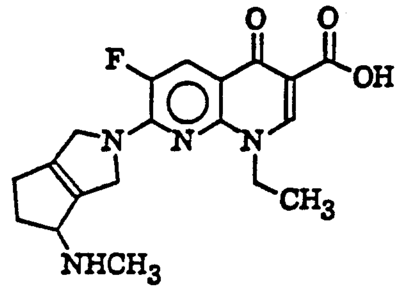

355mg of 6-methylamino-3-azabicyclo[3,3,0]oct-1(5)ene dihydrobromide and 270mg of 1-ethyl-7-chloro-6-fluoro-4-oxo-1,8-naphthyridine-3-carboxylic acid were subjected to the same process as described in Example 1 to obtain 231mg of the desired compound (yield: 62%).
m.p.: 185~189 °C

$^1$H-NMR ($CDCl_3$ + $CD_3COOD$, δ ppm) ; 8.74 (1H, s), 8.07 (1H, d, J = 12.4 Hz), 4.76~4.36 (7H, m), 2.71 (3H, s), 2.90~2.44 (4H, m), 1.51 (3H, t, J = 7 Hz)

Example 32: Preparation of 7-[6-amino-3-azabicyclo[3,3,0]oct-1(5) ene-3-yl]-1-(t-butyl)-6-fluoro-1,4-dihydro-4-oxoquinoline-3-carboxylic acid

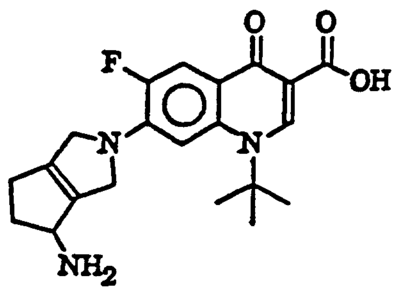

320mg of 6-amino-3-azabicyclo[3,3,0]oct-1(5)ene dihydrobromide and 281mg of 1-(t-butyl)-6,7-difluoro-1,4-dihydro-4-oxoquinoline-3-carboxylic acid were subejcted to the same process as described in Example 1 to obtain 205mg of the desired compound (yield: 53%).
m.p.: 193~196 °C

$^1$H-NMR ($CDCl_3$ + $CD_3COOD$, δ ppm) ; 8.55 (1H, s), 7.83 (1H, d, J = 12.3 Hz), 7.21 (1H, s), 4.70~4.49 (5H, m), 2.85~2.43 (4H, m), 1.91 (9H, s)

Example 33: Preparation of 7-[6-methylamino-3-azabicyclo[3,3,0]oct-1(5)ene-3-yl]-1-(t-butyl)-6-fluoro-1,4-dihydro-4-oxoquinoline-3-carboxylic acid

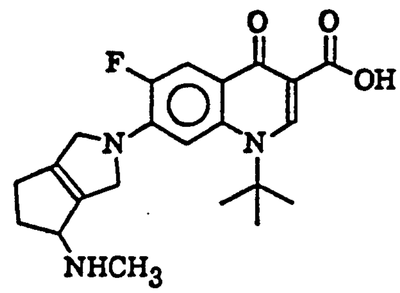

350mg of 6-methylamino-3-azabicyclo[3,3,0]oct-1(5)ene dihydrobromide and 281mg of 1-(t-butyl)-6,7-difluoro-1,4-dihydro-4-oxoquinoline-3-carboxylic acid were subjected to the same process as described in Example 1 to obtain 248mg of the desired compound (yield: 62%).
m.p.: 198~202°C

$^1$H-NMR ($CDCl_3$ + $CD_3COOD$, δ ppm) ; 8.82 (1H, s), 7.81 (1H, d, J = 12.3 Hz), 7.19 (1H, s), 4.70~4.48 (5H, m), 2.85~2.40 (4H, m), 2.72 (3H, s), 1.90 (9H, s)

Example 34: Preparation of 7-[6-acetamino-3-azabicyclo[3,3,0]oct-1(5)ene-3-yl]-1-cyclopropyl-6,8-difluoro-1,4-dihydro-4-oxoquinoline-3-carboxylic acid

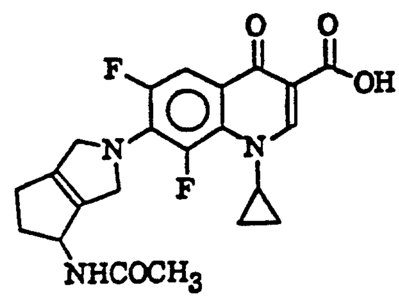

193mg of 7-[6-amino-3-azabicyclo[3,3,0]oct-1(5)ene-3-yl]-1-cyclopropyl-6,8-difluoro-1,4-dihydro-4-oxoquinoline-3-carboxylic acid was dissolved in a mixture of 3mℓ of pyridine and 2.5mℓ of acetic anhydride; and the resulting solution was stirred at a room temperature over night. The reaction mixture was poured into 20mℓ of water and stirred at a room temperature for 2 hours. The precipitate produced was filtered, washed with water, isopropanol and ethylether and dried under a reduced pressure to obtain 174mg of the desired compound(yield: 90%).
m.p.: 199~203°C

$^1$H-NMR ($CDCl_3$ + $CD_3COOD$, δ ppm) ; 8.77 (1H, s), 7.78 (1H, d, J = 14.6 Hz), 4.60 (4H, br, s), 4.50 (1H, m), 4.05 (1H, m), 2.91 (1H, m), 2.60 (1H, m), 2.52 (1H, m), 2.35 (1H, m), 2.01 (3H, s), 1.26 (4H, m)

Example 35: Preparation of 7-[6-t-butoxycarbonylamino-3-azabicyclo-[3,3,0]oct-1(5)ene-3-yl]-1-cyclopropyl-6,8-difluoro-1,4-dihydro-4-oxoquinoline-3-carboxylic acid methoxymethylester

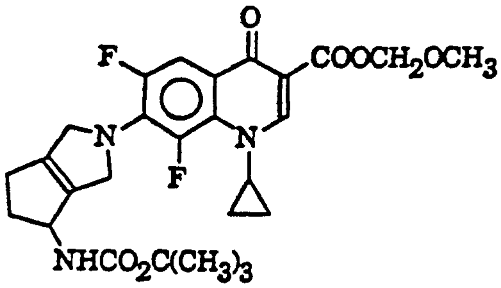

487mg of 7-[6-t-butoxycarbonylamino-3-azabicyclo[3,3,0] oct-1(5)ene-3-yl]-1-cyclopropyl-6,8-difluoro-1,4-dihydro-4-oxoquinoline-3-carboxylic acid was suspended in a mixture of 10mℓ of hexamethylphosphoramide and 5mℓ of tetrahydrofuran. To the resulting solution were added 250mg of methoxymethyliodide and 200mg of potassium carbonate powder; and the reaction mixture was stirred at a room temperature for 24 hours followed by an addition of 50mℓ of dichloromethane. The resultant was washed with water and 5% $NaHCO_3$; and the organic layer was then dehydrated with anhydrous sodium sulfate and concentrated. The residue thus obtained was crystallized with chloroform-hexane to obtain 298mg of the desired compound (yield: 56%).
m.p.: 155~158 °C

$^1$H-NMR ($CDCl_3$, δ ppm) ; 8.79 (1H, S), 7.75 (1H, d, J = 14.5 Hz), 4.62 (4H, br, s), 4.51 (1H, m), 4.25 (2H, br, s), 4.06 (1H, m), 3.81 (3H, s), 2.90 (1H, m), 2.61 (1H, m), 2.54 (1H, m), 2.33 (1H, m), 1.92 (9H, s), 1.25~1.15 (4H, m)

Example 36: Preparation of 7-[6-amino-3-azabicyclo[3,3,0]oct-1(5) ene-3-yl]-6,8-difluoro-1,4-dihydro-4-oxoquinoline-3-carboxylic acid lactate

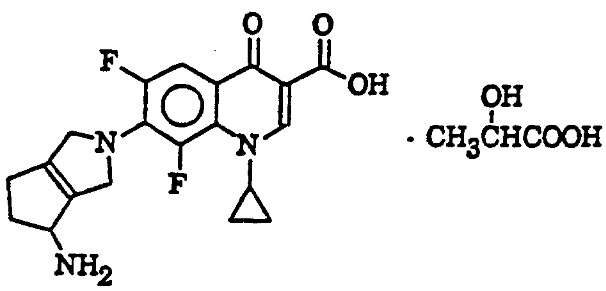

387mg of 7-[6-amino-3-azabicyclo[3,3,0]oct-1(5)ene-3-yl]-6,8-difluoro-1,4-dihydro-4-oxoquinoline-3-carboxylic acid was dissolved in a mixture of 10mℓ of dichloromethane and 2mℓ of ethanol; and the resulting solution was stirred at a room temperature for 2 hours after an addition of 87μℓ of 85% lactic acid. The solvent was evaporated under a reduced pressure to obtain quantitatively the desired compound.
m.p.: 180~184 °C

$^1$H-NMR ($CD_3OD + CD_3COOD$, δ ppm) ; 8.74 (1H, s), 7.72 (1H, d, J = 14 Hz), 4.8~4.1 (7H, m), 2.85~2.35 (4H, m), 1.50~1.16 (7H, m)

Use Example 1

A capsule formulation was prepared in accordance with the following composition:

| Component | Amount |
|---|---|
| Compound prepared in Example 1 | 100.0mg |
| corn starch | 25.0mg |
| calcium carboxymethyl cellulose | 23.0mg |
| magnesium stearate | 2.0mg |
| total | 150.0mg |

Use Example 2

A solution formulation was prepared in accordance with the following composition:

| Compoment | Amount |
|---|---|
| Compound prepared in Example 36 | 1 to 10g |
| lactic acid or sodium hydroxide | 0.1 to 2g |
| mannitol | 0.1g |
| deionized water | 87.9 to 98.8g |
| total | 150g |

1. In vitro anti-bacterial activity test

In order to demonstrate the superior effectiveness of the quinolone derivatives of the present invention, the minimal inhibitory concentration(MIC) of several compounds synthesized in Examples hereof against the standard strains was determined and compared with ofloxacin and ciprofloxacin. These MIC values were taken by employing two-fold dilution method: that is, two-fold serial dilutions of each of the test compounds were made and dispersed in a Mueller-Hinton agar medium; a standard strain which had the value of $10^7$ CFU/mℓ was inoculated on the medium, which was then incubated at 37°C for 18 hours. The results of the MIC tests are shown in Table 1.

Table 1. In vitro anti-bacterial activity test(MIC: µg/mℓ)

| No | Strain | Compound prepared in Example 1 | Compound prepared in Example 2 | Compound prepared in Example 3 | Compound prepared in Example 4 |
|---|---|---|---|---|---|
| 1 | Streptococcus pyogenes 308A | 0.391 | 0.195 | 0.195 | 0.195 |
| 2 | Streptococcus pyogenes 77A | 0.098 | 0.098 | 0.049 | 0.025 |
| 3 | Streptococcus faecium MD 8b | 0.098 | 0.098 | 0.098 | 0.049 |
| 4 | Staphylococcus aureus SG 511 | 0.098 | 0.049 | 0.025 | 0.025 |
| 5 | Staphylococcus aureus 285 | 0.098 | 0.098 | 0.049 | 0.049 |
| 6 | Staphylococcus aureus 503 | 0.049 | 0.049 | 0.049 | 0.025 |
| 7 | Escherichia coli O 55 | 0.025 | 0.013 | 0.049 | 0.025 |
| 8 | Escherichia coli DC 0 | 0.391 | 0.781 | 0.781 | 0.781 |
| 9 | Escherichia coli DC 2 | 0.098 | 0.098 | 0.049 | 0.025 |
| 10 | Escherichia coli OTEM | 0.049 | 0.098 | 0.098 | 0.098 |
| 11 | Escherichia coli 1507E | 0.049 | 0.098 | 0.098 | 0.098 |
| 12 | Pseudomonas aeruginosa 9027 | 1.563 | 3.125 | 1.563 | 3.125 |
| 13 | Pseudomonas aeruginosa 1592E | 1.563 | 1.563 | 1.563 | 3.125 |
| 14 | Pseudomonas aeruginosa 1771 | 1.563 | 1.563 | 1.563 | 3.125 |
| 15 | Pseudomonas aeruginosa 1771M | 0.391 | 0.781 | 0.391 | 0.781 |
| 16 | Salmonella typhimurium | 0.049 | 0.391 | 0.049 | 0.049 |
| 17 | Klebsiella aerogenes 1082E | 0.007 | 0.013 | 0.013 | <0.002 |
| 18 | Klebsiella aerogenes 1522E | 0.098 | 0.049 | 0.195 | 0.195 |
| 19 | Enterobacter cloacae P99 | 0.098 | 0.049 | 0.098 | 0.098 |
| 20 | Enterobacter cloacae 1321E | 0.025 | 0.013 | 0.098 | 0.098 |

Table 1(Continued)

| No | Strain | Compound prepared in Example 9 | Compound prepared in Example 10 | Compound prepared in Example 11 | Compound prepared in Example 15 |
|---|---|---|---|---|---|
| 1 | Streptococcus pyogenes 308A | 0.391 | 0.195 | 0.391 | 0.781 |
| 2 | Streptococcus pyogenes 77A | 0.098 | 0.049 | 0.195 | 0.195 |
| 3 | Streptococcus faecium MD 8b | 0.098 | 0.195 | 0.195 | 0.195 |
| 4 | Staphylococcus aureus SG 511 | 0.098 | 0.025 | 0.195 | 0.098 |
| 5 | Staphylococcus aureus 285 | 0.098 | 0.049 | 0.049 | 0.195 |
| 6 | Staphylococcus aureus 503 | 0.098 | 0.025 | 0.049 | 0.098 |
| 7 | Escherichia coli O 55 | 0.049 | 0.025 | 0.025 | 0.049 |
| 8 | Escherichia coli DC 0 | 0.781 | 0.781 | 1.563 | 1.563 |
| 9 | Escherichia coli DC 2 | 0.195 | 0.049 | 0.049 | 0.391 |
| 10 | Escherichia coli OTEM | 0.098 | 0.049 | 0.049 | 0.098 |
| 11 | Escherichia coli 1507E | 0.098 | 0.049 | 0.098 | 0.195 |
| 12 | Pseudomonas aeruginosa 9027 | 3.125 | 1.563 | 3.125 | 3.125 |
| 13 | Pseudomonas aeruginosa 1592E | 3.125 | 1.563 | 3.125 | 3.125 |
| 14 | Pseudomonas aeruginosa 1771 | 3.125 | 1.563 | 3.125 | 3.125 |
| 15 | Pseudomonas aeruginosa 1771M | 0.781 | 0.781 | 1.563 | 1.563 |
| 16 | Salmonella typhimurium | 0.013 | 0.025 | 0.098 | 0.195 |
| 17 | Klebsiella aerogenes 1082E | 0.195 | 0.007 | 0.007 | 0.025 |
| 18 | Klebsiella aerogenes 1522E | 0.195 | 0.098 | 0.195 | 0.195 |
| 19 | Enterobacter cloacae P99 | 0.049 | 0.025 | 0.195 | 0.049 |
| 20 | Enterobacter cloacae 1321E | | 0.025 | 0.049 | 0.049 |

Table 1(Continued)

| No | Strain | Compound prepared in Example 17 | Ofloxacin | Cipro-floxacin |
|---|---|---|---|---|
| 1 | Streptococcus pyogenes 308A | 3.125 | 3.125 | 3.125 |
| 2 | Streptococcus pyogenes 77A | 0.195 | 1.563 | 0.781 |
| 3 | Streptococcus faecium MD 8b | 0.391 | 0.781 | 0.781 |
| 4 | Staphylococcus aureus SG 511 | 0.391 | 0.391 | 0.781 |
| 5 | Staphylococcus aureus 285 | 0.781 | 0.391 | 0.781 |
| 6 | Staphylococcus aureus 503 | 0.781 | 0.391 | 0.781 |
| 7 | Escherichia coli O 55 | 0.195 | 0.025 | 0.007 |
| 8 | Escherichia coli DC 0 | 3.125 | 0.781 | 0.391 |
| 9 | Escherichia coli DC 2 | 0.391 | 0.391 | 0.195 |
| 10 | Escherichia coli OTEM | 0.391 | 0.049 | 0.013 |
| 11 | Escherichia coli 1507E | 0.781 | 0.098 | 0.025 |
| 12 | Pseudomonas aeruginosa 9027 | 12.500 | 1.563 | 0.781 |
| 13 | Pseudomonas aeruginosa 1592E | 6.250 | 1.563 | 0.391 |
| 14 | Pseudomonas aeruginosa 1771 | 12.500 | 1.563 | 0.781 |
| 15 | Pseudomonas aeruginosa 1771M | 3.125 | 0.391 | 0.195 |
| 16 | Salmonella typhimurium | 0.391 | 0.098 | 0.049 |
| 17 | Klebsiella aerogenes 1082E | 0.195 | 0.025 | 0.025 |
| 18 | Klebsiella aerogenes 1522E | 1.563 | 0.098 | 0.013 |
| 19 | Enterobacter cloacae P99 | 0.781 | 0.098 | 0.025 |
| 20 | Enterobacter cloacae 1321E | 0.391 | 0.049 | 0.007 |

2. Preventive effect on the systemic infection

10 fold of the pathogen which leads to 100% lethality were injected intraperitoneally to male and female NMRI mice weighing 18 to 20g. Immediately and after 4 hours, the dose of test compounds which was determined by two-fold serial dilution method was administered orally or subcutaneously; and on day 7, the effectiveness was evaluated in terms of $ED_{50}$ calculated from the number of survived mice by the probit analysis.

Table 2

| Preventive effect on the systemic infection | | | |
|---|---|---|---|
| Pathogen | Compound | $ED_{50}$: mg/kg | |
| | | Subcutaneous Injection | Oral Administration |
| *S. aureus* 17740 | Compound prepared in Example 1 | 25 | 105 |
| | Compound prepared in Example 2 | 27 | 112 |
| | Ofloxacin | 200.0 | >200.0 |
| | Ciprofloxacin | 132.3 | >200.0 |
| *P. mirabilis* | Compound prepared in Example 1 | 1.55 | 5.5 |
| | Compound prepared in Example 3 | 1.43 | 5.9 |
| | Ofloxacin | 0.53 | 2.27 |
| | Ciprofloxacin | 0.28 | 0.92 |

3. Acute Toxicity Test

Test compounds were administered to female ICR mice weighing 20 to 25g and on day 14, $LD_{50}$ was calculated from the number of survived mice by the probit analysis.

Table 3

| Result of the Acute Toxicity Test | | |
|---|---|---|
| Compound | Acute Toxicity($LD_{50}$: mg/kg) | |
| | Intraperitoneal Injection | Oral Administration |
| Compound prepared in Example 1 | 720 | 4000 |
| Compound prepared in Example 2 | 810 | 4100 |
| Compound prepared in Example 3 | 731 | 4100 |
| Compound prepared in Example 6 | 755 | 4300 |

As can be seen from the results, the compounds of the present invention possess a broad spectrum of potent anti-bacterial activities against gram-positive and-negative bacteria as compared with the known quinolone antibiotics, ciprofloxacin and ofloxacin. The compounds of the present invention also exhibit superior activities to the known quinolone antibiotics in terms of 50% effective dose($ED_{50}$) on the systemic bacterial infection. Further, the compounds of the present invention have low toxicity sufficient to be proved as drugs.

Accordingly, the compounds of the present invention will be useful as therapeutical compounds and preservatives of inorganic and organic material.

## Claims

**1.** A quinolone carboxylic acid derivative of formula(I) and pharmaceutically acceptable salts thereof:

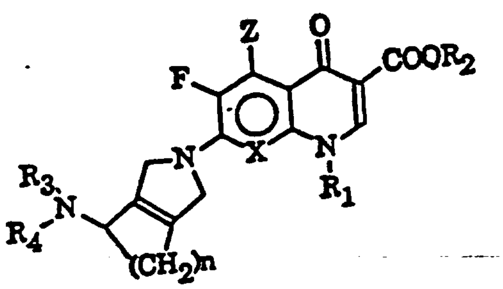

(I)

wherein:

| | |
|---|---|
| X is | a nitrogen atom or a C-Y group wherein Y is a hydrogen, fluorine, chlorine or bromine atom or a methoxy or methyl group; |
| $R_1$ is | a $C_{1-6}$ alkyl group optionally substituted with a halogen atom or a hydroxy radical, an alkenyl group, a $C_{3-6}$ cycloalkyl group, a phenyl group substituted with a halogen atom or a divalent group of $-OCH_2{}^*CH(CH_3)-$, $-SCH_2{}^*CH_2-$or $-SCH_2{}^*CH(CH_3)-$ which forms an oxazine or thiazine ring together with the nitrogen atom to which $R_1$ is attached and with X wherein X is C-Y; |
| $R_2$ is | a hydrogen atom, a carboxy protecting group or a pharmaceutically acceptable metal or organic cation; |
| $R_3$ and $R_4$, | which may be the same or different, are a hydrogen atom, a lower alkyl group, an acyl group or a nitrogen protecting group metabolizable in vivo; |
| Z is | a hydrogen or halogen atom, an amino, hydroxy or methyl group; and |
| n is | 1 to 3. |

2. The compound of claim 1 wherein a carboxy protecting group is a lower alkyl, lower alkanoyloxy-lower alkyl, lower alkoxycarbonyloxy-lower alkyl, lower alkoxymethyl, di(lower alkyl) amino-lower alkyl or 4-methylene-5-methyl-1,3-dioxolene-2-one group.

3. The compound of claim 1 which is the salt with a hydrochloric, sulfuric, phosphoric, nitric, methanesulfonic, acetic, formic, propionic, lactic, maleic, malonic, fumaric, tataric, citric, paratoluenesulfonic, ascorbic or glutamic acid.

4. A process for preparing a compound of formula(Ia), which comprises reacting a compound of formula(II) with a compound of formula(III) or (IIIa)

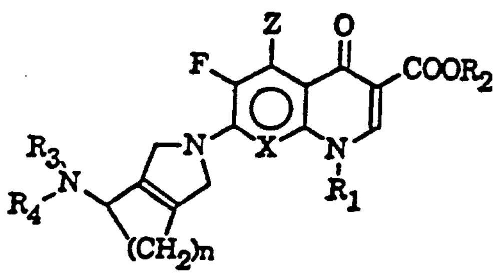

(Ia)

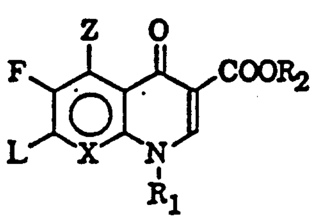

(II)

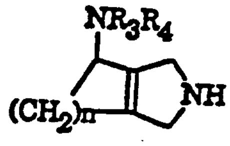

(III)

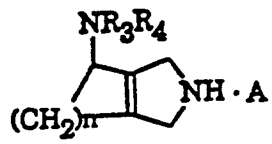

(IIIa)

whrein:

| | |
|---|---|
| $R_1$, $R_3$, $R_4$, X, Z and n are | the same as defined in claim 1; |
| $R_2$ is | a hydrogen atom or a carboxy protecting group as defined in claim 2; |
| L is | a leaving group which is a fluorine, chlorine or bromine atom, a methanesulfonyl group or a para-toluenesulfonyl group; and |
| A is | a hydrochloric, bromic, sulfuric or trifluoroacetic acid. |

**5.** A process for preparing a compound of formula(Ia-2) wherein $R_2$ is carboxy protecting group, which comprises reacting a compound of formula(Ia-1) with a compound of $R_2$-Hal:

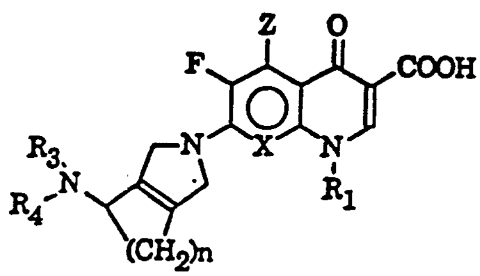

(Ia-1)

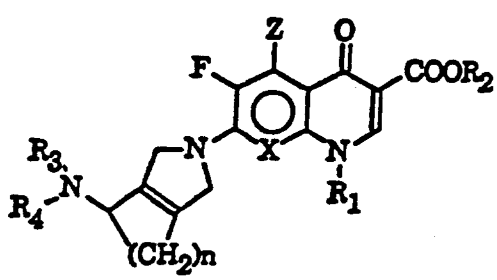

(Ia-2)

wherein:

| | |
|---|---|
| $R_1$, $R_3$, $R_4$, X, Z and n are | the same as defined in claim 1; |
| $R_2$ is | a carboxy protecting group as defined in claim 2; and |
| Hal is | a chlorine, bromine or iodine atom. |

**6.** A process for preparing a compound of formula(Ia-3) wherein $R_2$ is a pharmaceutically acceptable metal or organic cation, which comprises reacting a compound of formula(Ia-1) with a compound of $R_2$-

donor:

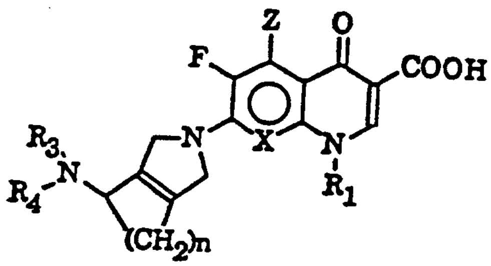

(Ia-1)

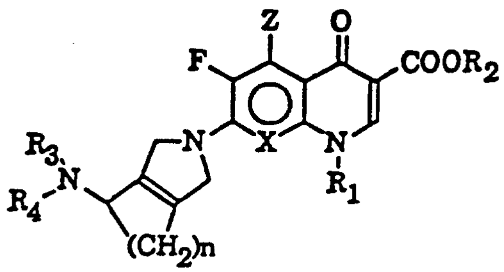

(Ia-3)

wherein:

| | |
|---|---|
| $R_1$, $R_3$, $R_4$, X, Z and n are | the same as defined in claim 1; and |
| $R_2$-donor is | sodium hydroxide, potassium hydroxide, calcium hydroxide, magnesium hydroxide, silver nitrate or tertiary or quarternary $C_{1-4}$ alkylammonium. |

7. A process for preparing a compound of formula(Ia-5), which comprises reacting a compound of formula(Ia-4) with a compound of $R_4$-donor:

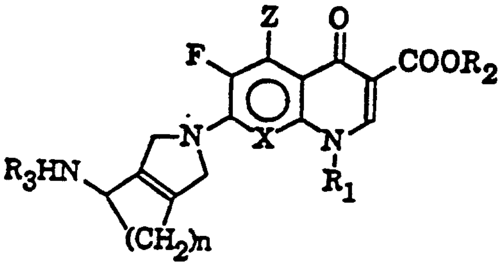

(Ia-4)

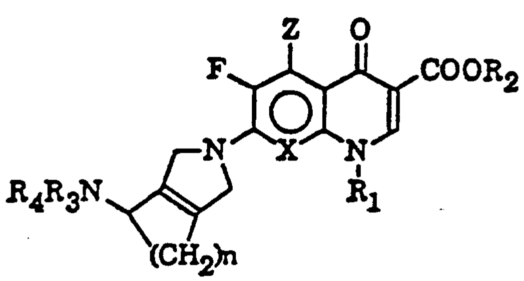

(Ia-5)

wherein:

| | |
|---|---|
| $R_1$, $R_3$, $R_4$, X, Z and n are | the same as defined in claim 1; |
| $R_2$ is | a hydrogen atom or a carboxy protecting group as defined in claim 2; and |
| $R_4$-donor is | $R_4$-Hal wherein Hal is a chlorine, bromine or iodine atom and $R_4$ is the |

same as defined in claim 1, excepting a hydrogen atom.

**8.** An antibacterial formulation comprising at least one of the compounds of formula(I) as an active component.

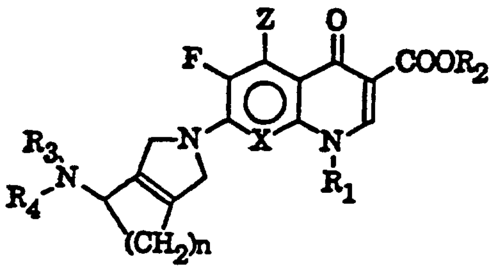

(I)

wherein:

$R_1$, $R_2$, $R_3$, $R_4$, X, Z and n are the same as defined in claim 1.

European Patent Office

# EUROPEAN SEARCH REPORT

Application Number

EP 92 11 6759

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 343 560 (WAKUNAGA SEIYAKU KABUSHIKI KAISHA)<br>* claims *<br>--- | 1,8 | C07D401/04<br>A61K31/40<br>C07D471/04<br>C07D498/06 |
| A | EP-A-0 429 304 (SHIONOGI SEIYAKU KABUSHIKI KAISHA)<br>* claims *<br>--- | 1,8 | |
| A | EP-A-0 343 524 (SHIONOGI SEIYAKU KABUSHIKI KAISHA)<br>* claims *<br>--- | 1,8 | |
| A | EP-A-0 424 850 (KOREA RESEARCH INSTITUTE OF CHEMICAL TECHNOLOGY)<br>* claims *<br>--- | 1,8 | |
| P,A | WO-A-9 212 146 (BANYU PHARMACEUTICAL CO., LTD.)<br>* claims *<br>----- | 1,8 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 17 NOVEMBER 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)